(19) **Europäisches Patentamt / European Patent Office / Office européen des brevets**

(11) **EP 2 822 451 B1**

(12) **FASCICULE DE BREVET EUROPEEN**

(45) Date de publication et mention de la délivrance du brevet:
**02.06.2021 Bulletin 2021/22**

(21) Numéro de dépôt: **13719887.5**

(22) Date de dépôt: **08.03.2013**

(51) Int Cl.:
*A61B 3/14* (2006.01)  *G06F 3/01* (2006.01)
*A61B 3/11* (2006.01)  *A61B 3/113* (2006.01)
*A61B 5/11* (2006.01)

(86) Numéro de dépôt international:
**PCT/FR2013/000060**

(87) Numéro de publication internationale:
**WO 2013/132165 (12.09.2013 Gazette 2013/37)**

(54) **PROCEDE DE DETERMINATION D'AU MOINS UNE CARACTERISTIQUE DE POSTURE DE LA TETE D'UN PORTEUR D'UNE PAIRE DE LUNETTES**

VERFAHREN ZUR BESTIMMUNG VON MINDESTENS EINER KOPFHALTUNGSEIGENSCHAFT EINER BRILLENTRAGENDEN PERSON

METHOD FOR DETERMINING AT LEAST ONE HEAD POSTURE CHARACTERISTIC OF A PERSON WEARING SPECTACLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.03.2012 FR 1200703**

(43) Date de publication de la demande:
**14.01.2015 Bulletin 2015/03**

(73) Titulaire: **Essilor International**
**94220 Charenton-le-Pont (FR)**

(72) Inventeurs:
• **HADDADI, Ahmed**
**94220 Charenton-le-Pont (FR)**
• **DELZERS, Jean**
**94220 Charenton le Pont (FR)**

(74) Mandataire: **Jacobacci Coralis Harle**
**32, rue de l'Arcade**
**75008 Paris (FR)**

(56) Documents cités:
DE-A1-102009 004 383   FR-A1- 2 860 887
US-A1- 2006 281 969   US-A1- 2009 109 400
US-A1- 2010 128 220   US-A1- 2010 208 206

**Description**

DOMAINE TECHNIQUE AUQUEL SE RAPPORTE L'INVENTION

La présente invention concerne de manière générale la prise de mesures sur un sujet.
Elle trouve une application particulière, mais non exclusive, à la prise de mesures sur un porteur de lunettes en vue de la conception optique personnalisée de lentilles correctrices adaptées à ce porteur.
Elle concerne plus particulièrement un procédé de détermination d'au moins une caractéristique de posture de la tête du porteur.
Elle concerne également une méthode d'acquisition des demi-écarts pupillaires du porteur et une méthode de détection du comportement du porteur lorsqu'il doit détourner rapidement le regard.
Elle concerne aussi un dispositif de détermination d'au moins une caractéristique de posture de la tête du porteur comprenant au moins une source de lumière adaptée à éclairer la tête du porteur de lunettes, un capteur d'images adapté à acquérir une image de la tête du porteur sur laquelle apparaît la paire de lunettes éclairée par la source de lumière, et une unité de calcul de ladite caractéristique de posture.

ARRIERE-PLAN TECHNOLOGIQUE

Lors de la conception d'une lentille ophtalmique correctrice, on cherche à prendre en compte un nombre important de paramètres géométrico-morphologiques individuels, dits de conception optique personnalisée, attachés au porteur et à la monture de lunettes sélectionnée, afin d'usiner la lentille de telle sorte qu'elle s'adapte au mieux au porteur.
Pour déterminer ces paramètres géométrico-morphologiques, l'opticien place la monture de lunettes sélectionnée sur le nez du porteur et réalise différentes opérations de mesure sur le porteur ainsi équipé. Ainsi peut-il notamment déterminer les demi-écarts pupillaires du porteur, c'est-à-dire les distances entre l'arête du nez du porteur et chacune des pupilles de ce dernier.
Ces mesures sont toutefois faussées lorsque le visage du porteur ne fait pas exactement face au capteur d'images.
On constate en particulier que lorsque la tête du porteur présente un angle de lacet non nul par rapport à l'axe optique, c'est-à-dire lorsque la tête du porteur est légèrement tournée vers la gauche ou vers la droite du capteur d'image, les mesures des demi-écarts pupillaires sont faussées d'environ 0,5 millimètre par degré d'angle de lacet.
Il est alors connu de déterminer cet angle de lacet pour corriger les paramètres géométrico-morphologiques mesurés.
On connaît notamment du document WO 2008/132356 un procédé de détermination de cet angle, qui consiste à équiper la monture de lunettes d'un système de repérage pour faciliter la localisation de la monture de lunettes sur l'image acquise et pour faciliter le calcul de l'angle de lacet.
Ce procédé présente toutefois divers inconvénients.
Il est ainsi nécessaire de fixer avec soin le système de repérage sur la monture de lunettes, quelle que soit la forme de cette monture, ce qui peut s'avérer fastidieux et qui nécessite d'être mis en oeuvre par un opticien formé.
La tenue du système de repérage sur la monture peut en outre se révéler aléatoire, selon la forme de la monture de lunettes.
On observe par ailleurs que la localisation du système de repérage est difficile à mettre en oeuvre automatiquement et présente un taux d'échec significatif, si bien que l'opticien est souvent forcé de localiser manuellement ce système de repérage sur l'image acquise.
Enfin, le système de repérage est peu esthétique, ce qui n'est pas flatteur pour le porteur de lunettes qui se voit dans un miroir et sur l'image acquise.
On connaît par ailleurs du document WO 2008/009423 un procédé pour détecter les positions de deux lentilles dans une monture de lunettes, qui utilise également un système de repérage et un capteur d'images. Le système de repérage consiste ici en des autocollants collés sur les lentilles, tandis que le capteur d'image est formé de deux caméras distinctes (ou une caméra et un miroir semi-transparent).
Deux sources de lumière situées au-dessus du porteur de lunettes sont prévues pour éclairer le porteur sans pour autant générer de reflets sur les lentilles qui perturberaient les mesures.
Ici également, ce procédé présente divers inconvénients.
Il est ainsi nécessaire de coller avec soin les autocollants sur les lentilles.
La localisation automatique des autocollants est difficile à mettre en œuvre et présente un taux d'échec significatif.
L'utilisation de deux caméras distinctes (ou une caméra et un miroir semi-transparent) nécessite par ailleurs de fabriquer avec une grande précision le dispositif de telle sorte que ces deux caméras soient précisément positionnées l'une par rapport à l'autre.

Elle nécessite en outre l'intervention récurrente de techniciens pour repositionner régulièrement les caméras.

La précision des mesures est en outre très sensible à la température, dont la hausse provoque la dilatation des éléments constitutifs du dispositif.

Le document DE102009004383 décrit pour sa part un dispositif et un procédé de détermination de caractéristiques de posture d'un porteur de lunettes.

Ce dispositif comporte trois néons, ainsi que deux lampes flash ou à incandescence. Ce dispositif comporte également deux caméras distinctes, ainsi qu'une unité de calcul.

Cette unité de calcul est adaptée à traiter en combinaison les images acquises par les deux caméras afin de déterminer les caractéristiques de posture de la tête du porteur par rapport aux caméras.

Il est mentionné que ces caractéristiques de posture peuvent être acquises à l'aide des reflets générés par les sources de lumière sur les bords des deux lentilles, de manière à en déduire des caractéristiques de posture tridimensionnelle, à l'aide d'un système stéréo (deux caméras).

OBJET DE L'INVENTION

Afin de remédier aux inconvénients précités de l'état de la technique, la présente invention propose un procédé de détermination d'au moins une caractéristique de posture de la tête d'un porteur d'une paire de lunettes équipée d'une monture et de deux lentilles, tel que défini dans la revendication 1.

Ainsi, grâce à l'invention, connaissant la position de la source de lumière par rapport au capteur d'images, on utilise les positions des reflets sur les faces optiques des lentilles pour déterminer la caractéristique de posture de la tête du porteur.

Ces reflets sont majoritairement réfléchis par les faces optiques avant des lentilles, mais ils peuvent également être en partie réfléchis par les faces optiques arrière des lentilles.

Il n'est donc nullement nécessaire de prévoir un quelconque système de repérage sur la paire de lunettes, si bien que le procédé peut être mis en œuvre simplement, sans l'aide d'un quelconque spécialiste.

L'utilisation d'une source de lumière et d'un simple capteur d'images (à l'exclusion de tout autre capteur d'images ou de miroir semi-réfléchissant) permet par ailleurs d'obtenir une grande fiabilité dans les mesures, puisque celles-ci sont peu sensibles aux variations de température.

Le dispositif, dont la fabrication est particulièrement simple, présente des coûts de fabrication et d'entretien réduits.

L'exploitation des positions des reflets sur les faces avant des deux lentilles, et non sur les bords des lentilles ou sur les bords de trous percés dans les lentilles, est particulièrement avantageuse.

En effet, si on exploitait les positions des reflets sur les bords des lentilles, on ne pourrait obtenir qu'une unique donnée d'orientation de la lentille par rapport à la source de lumière (on comprend en effet qu'il est possible de bouger la source de lumière par rapport à la lentille sans modifier la position du reflet sur le bord de la lentille).

Ici, on peut au contraire obtenir deux donnés relatives à l'orientation horizontale et à l'orientation verticale de la lentille par rapport à la source de lumière, ce qui permet d'en déduire des informations tridimensionnelles de posture du porteur (on comprend en effet qu'il est impossible de bouger la source de lumière par rapport à la lentille sans modifier la position du reflet sur la face avant de la lentille).

D'autres caractéristiques avantageuses et non limitatives du procédé de détermination conforme à l'invention sont définies dans les revendications 1 à 6.

La présente invention propose également une méthode d'acquisition des demi-écarts pupillaires d'un porteur d'une paire de lunettes, telle que définie dans la revendication 7.

D'autres caractéristiques avantageuses et non limitatives de la méthode d'acquisition conforme à l'invention sont définies dans les revendications 8 à 11.

La présente invention propose aussi une méthode de détection du comportement d'un porteur d'une paire de lunettes à l'aide d'un dispositif comprenant un capteur d'images, au moins une source de lumière, deux cibles et une unité de calcul, telle que définie en revendication 12.

D'autres caractéristiques avantageuses et non limitatives de la méthode de détection sont définies en revendications 13 et 14.

L'invention concerne enfin un dispositif de détermination d'au moins une caractéristique de posture de la tête d'un porteur d'une paire de lunettes, tel que défini en revendication 15.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 est une vue schématique en perspective de la tête d'un porteur de lunettes ;
- la figure 2 est une vue schématique de dessus de la tête du porteur de lunettes de la figure 1 ;
- la figure 3 est un schéma sur lequel apparaissent les yeux du porteur de lunettes de la figure 1 et un capteur d'images ;
- la figure 4 est une vue schématique en perspective d'un dispositif adapté à mettre en oeuvre le procédé selon l'invention ;
- les figures 5 à 7 sont des représentations de trois images acquises par le capteur d'images de la figure 3 ; et
- la figure 8 est une vue schématique de dessus de la tête du porteur de lunettes de la figure 1.

Dans la description qui va suivre, certaines références auront un numéro suivi de la lettre G ou D. Ces références désigneront alors respectivement des éléments situés à gauche ou à droite par rapport au porteur de lunettes. Les yeux gauche et droit du porteur seront ainsi respectivement référencés 20G et 20D.

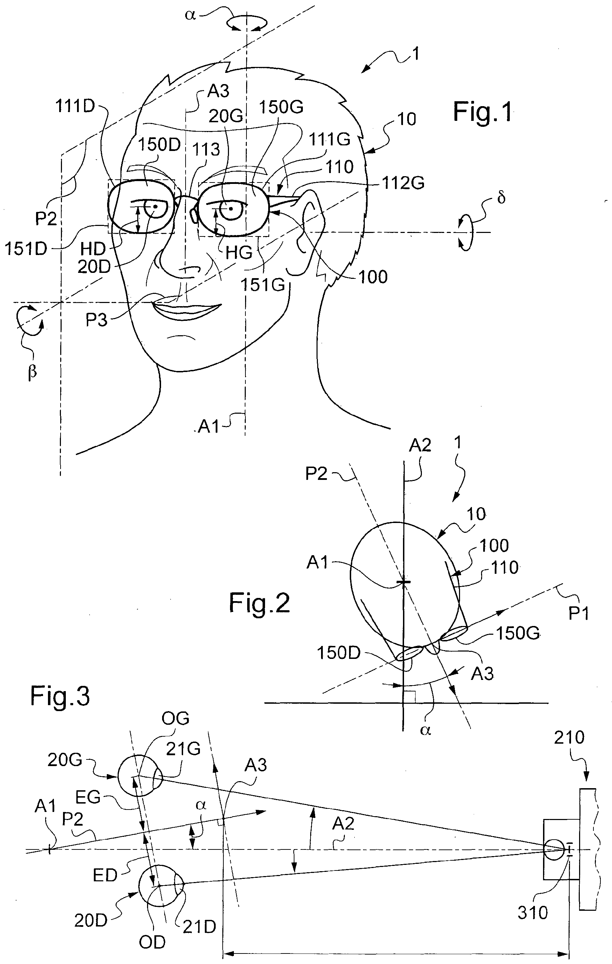

Sur la figure 1, on a représenté la tête 10 d'un individu 1 portant une paire de lunettes 100. Cet individu sera désigné dans la suite de cet exposé comme le « porteur ».

Dans cet exposé, on considérera également un autre individu, qui sera désigné comme le « vendeur », et qui aidera le porteur 10 à choisir une paire de lunettes 100 et à mesurer les données nécessaires à la fabrication des lentilles ophtalmiques (non représentées) en vue de leur commande et de leur montage dans la monture de lunettes 100 sélectionnée par le porteur.

Grâce à l'automatisation des mesures, ce vendeur ne sera pas nécessairement un opticien.

Préalablement aux mesures, le porteur choisira une paire de lunettes 100 parmi les paires de lunettes mises à disposition par le vendeur.

Lors des mesures, le porteur portera cette paire de lunettes 100.

Il sera positionné dans une configuration assise ou debout, avec sa tête 10 sensiblement droite et dirigée vers un capteur d'images 210 (voir figure 3).

Le vendeur demandera par ailleurs au porteur 1 de regarder une cible 310 située à proximité du capteur d'images 210 (voir figure 3). Les centres des deux pupilles 21G, 21D et les centres de rotation OD, OG des deux yeux 20G, 20D du porteur 1 seront donc respectivement alignés avec la cible 310.

Sur la figure 1, on observe que la paire de lunettes 100 choisie par le porteur 1 est du type cerclée (en variante, elle pourrait bien entendu être d'une autre sorte, telle que semi-cerclée ou percée).

Cette paire de lunettes 100 comporte une monture de lunettes 110, avec deux cercles 111G, 111D (ou entourages) dans lesquels sont emboîtées deux lentilles de présentation 150G, 150D (destinées à être remplacées par les lentilles ophtalmiques adaptées à l'acuité visuelle du porteur).

Les deux cercles 111G, 111D sont reliés l'un à l'autre par un pont ou pontet 113 équipé de deux plaquettes nasales reposant sur le nez du porteur. Ils sont également chacun équipés d'une branche 112G, 112D reposant sur les oreilles correspondantes du porteur 1. Ces branches 112G, 112D s'étendent chacune de manière rectiligne sur leur majeure partie, selon un axe longitudinal, et sont recourbées à leurs extrémités.

Chacun des deux cercles 111G, 111D de la monture de lunettes 110 présente, en creux dans son flanc intérieur, une rainure communément appelée drageoir dans laquelle est emboîté un biseau qui s'étend en saillie de la tranche de la lentille de présentation 150G, 150D correspondante.

Comme le montre la figure 2, on définit ici par rapport à la monture de lunettes 110 un plan moyen P1 qui passe au plus près de l'ensemble des points de l'arête de fond des drageoirs des deux cercles 111G, 111D.

Ce plan moyen P1 est incliné par rapport au plan passant par les axes longitudinaux des branches 112G, 112D d'un angle appelé «angle pantoscopique ». L'angle pantoscopique moyen d'une monture de lunettes est de l'ordre de 10 degrés.

Comme le montre la figure 1, sur chaque image du porteur 1 acquise par le capteur d'images 210, on peut repérer les cercles 111G, 111D de la monture de lunettes 110 au moyen de deux cadres appelés « cadres boxing » 151G, 151D.

Ces cadres boxing 151G, 151D sont définis comme les rectangles circonscrits aux cercles 111G, 111D de la monture de lunettes 110 (ou aux lentilles de présentation 150G, 150D), dont deux des côtés sont verticaux et dont les deux autres sont horizontaux.

On définit alors sur chaque image un axe remarquable A3 de la monture de lunettes 100, comme étant l'axe qui est parallèle aux côtés verticaux des cadres boxing 151G, 151D, et qui est situé à égale distance de ces derniers.

Comme le montre la figure 1, on définit par rapport au porteur 1 en position de mesure un plan de Francfort P3 comme étant le plan passant par les points orbitaires inférieurs et le porion du porteur (le porion étant le point du crâne le plus élevé du conduit auditif, qui correspond au tragion de l'oreille). En position de mesure, ce plan de Francfort P3 sera donc sensiblement horizontal.

On définit également un plan sagittal P2 comme étant le plan orthogonal au plan de Francfort P3 et à l'axe passant par les centres de rotation OG, OD des deux yeux 20G, 20D du porteur 1, passant par l'arête du nez du porteur. Lors des mesures, ce plan sagittal P2 sera donc sensiblement vertical. Sa position sera déduite non pas de la position du nez du porteur 1, mais plutôt en fonction de la position de la paire de lunettes 100 portée par le porteur 1.

On définit par rapport à la tête 10 du porteur 1 un axe longitudinal A1 comme étant orthogonal au plan de Francfort P3, compris dans le plan sagittal P2, et correspondant à l'axe de rotation de la tête 10 du porteur 1 lorsque ce dernier fait un mouvement de tête de droite à gauche (c'est-à-dire lorsque le porteur fait Non de la tête). En position de mesure, cet axe longitudinal A1 sera sensiblement vertical.

On définit également un axe frontal A4 comme étant l'axe d'intersection entre le plan de Francfort P3 et le plan sagittal P2.

On définit aussi un axe transversal A5 comme étant l'axe perpendiculaire aux axes longitudinal A1 et frontal A4.

En position de mesure, ces deux axes frontal et transversal A4, A5 seront sensiblement horizontaux.

Comme le montre la figure 3, les demi-écarts pupillaires EG, ED du porteur 1 sont définis comme les distances séparant le plan sagittal P2 des centres de rotation OG, OD des deux yeux 20G, 20D du porteur 1.

Comme le montre la figure 1, les hauteurs pupillaires HG, HD du porteur sont quant à elles définies comme les distances séparant, sur chaque image acquise, les centres de rotation OG, OD des yeux 20G, 20D du porteur 1 (qui sont en pratique confondus sur l'image avec les centres des pupilles 21G, 21D du porteur 1) et le bord inférieur du cadre boxing 151G, 151D correspondant.

Tel que représenté sur la figure 3, on définit par ailleurs par rapport au capteur d'images 210 un plan horizontal et un plan vertical, dont l'intersection est confondue avec l'axe optique A2 du capteur d'images 210.

Idéalement, on cherche alors à placer la tête 10 du porteur 1 de manière que son plan de Francfort P3 se confonde avec le plan horizontal du capteur d'images 210 et que son plan sagittal P2 se confonde avec le plan vertical du capteur d'images 210.

En pratique, on constate généralement un léger décalage entre ces plans, susceptible de fausser les mesures.

Ce décalage peut être mesuré au moyen de trois angles de roulis $\beta$, de tangage $\delta$ et de lacet $\alpha$, qui correspondent aux trois mobilités de pivotement de la tête 10 du porteur 1.

L'angle de lacet $\alpha$ sera ainsi défini comme l'angle de pivotement de la tête 10 du porteur 1 autour de l'axe longitudinal A1, entre la position idéale de la tête 10 du porteur (face à l'axe optique A2) et la position réelle de la tête 10 du porteur. Cet angle de lacet $\alpha$ sera mesuré dans le plan horizontal du capteur d'images 210.

L'angle de roulis $\beta$ sera défini comme l'angle de pivotement de la tête 10 du porteur 1 autour de l'axe frontal A4, entre la position idéale de la tête 10 du porteur et la position réelle de la tête 10 du porteur. Cet angle de roulis $\beta$ sera facilement mesurable sur chaque image acquise, en fonction de l'inclinaison de l'axe remarquable A3.

L'angle de tangage $\delta$ sera défini comme l'angle de pivotement de la tête 10 du porteur 1 autour de l'axe transversal A5, entre la position idéale de la tête 10 du porteur (face à l'axe optique A2) et la position réelle de la tête 10 du porteur. Cet angle de tangage $\delta$ sera mesuré dans le plan vertical du capteur d'images 210.

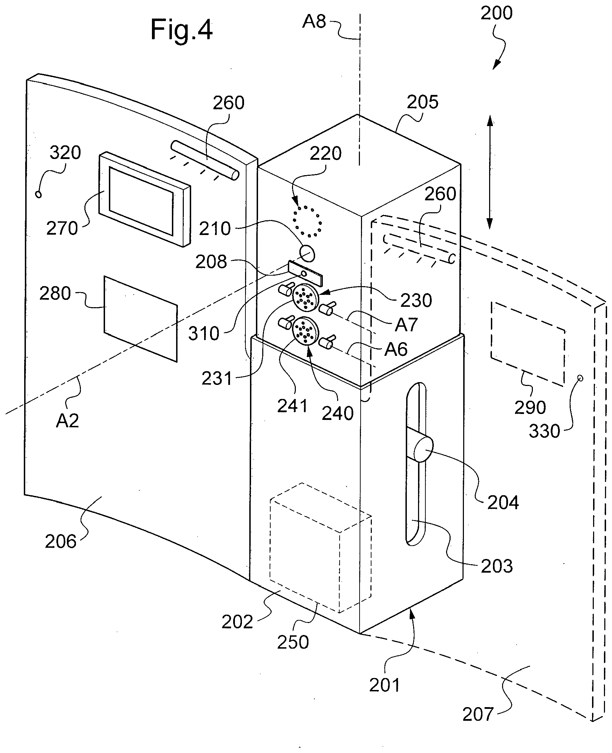

Sur la figure 4, on a représenté le dispositif 200 qui permet de mettre en œuvre le procédé selon l'invention et qui, plus généralement, permet de mesurer et d'acquérir les données nécessaires à la commande des lentilles ophtalmiques à monter dans la monture de lunettes 110 sélectionnée par le porteur 1, en lieu et place des lentilles de présentation 150G, 150D.

Ce dispositif se présente sous la forme d'un kiosque à lunettes 200, faisant simultanément office de présentoir de montures de lunettes et de centre de mesure.

Il comporte à cet effet au moins un châssis 201, une source de lumière 220, 230, 240, un capteur d'images 210 et une unité de calcul 250.

De manière préférentielle, la source de lumière 220, 230, 240 émet dans l'infrarouge et le capteur d'images 210 capte des images infrarouges.

Le domaine de l'infrarouge qui sera ici utilisé est le proche infrarouge, dont les longueurs d'onde sont comprises entre 780 et 1400 nm.

L'utilisation d'une lumière infrarouge présente en effet plusieurs avantages. Elle permet notamment de s'affranchir des reflets parasites, provenant de la lumière extérieure ou de la lumière intérieure qui se réfléchit sur les lentilles de présentation 150G, 150D de la paire de lunettes 100. Elle permet aussi d'éviter au porteur 1 d'être ébloui lors des mesures. Elle permet enfin de voir les yeux du porteur 1 sur les images acquises par le capteur d'images 210, même lorsque les lentilles de présentation 150D, 150G sont teintées.

Le châssis pourrait être réalisé d'une seule pièce, de telle manière que le capteur d'images et les sources de lumière soient immobiles par rapport au sol sur lequel le kiosque à lunettes repose. Dans cette éventualité, il serait nécessaire de prévoir un tabouret réglable en hauteur, de manière à pouvoir placer le porteur de telle sorte que sa tête entre dans le champ du capteur d'images.

Au contraire, dans le mode de réalisation du kiosque 200 représenté sur la figure 4, le châssis 201 comprend un pied 202 qui est posé sur le sol, et une coulisse 205 qui est montée mobile en translation sur le pied 202 suivant un axe vertical A8 et qui supporte les sources de lumière 220, 230, 240 et le capteur d'images 210.

La position du capteur d'images 210 est ainsi réglable en hauteur en fonction de la taille du porteur 1.

Le pied 202 présente plus précisément une forme de parallélépipède creux, allongé suivant l'axe vertical A8 et de section horizontale carrée.

Ce pied 202 présente une extrémité inférieure fermée par une paroi posée au sol, et une extrémité supérieure ouverte par laquelle s'engage la coulisse 205.

Cette coulisse 205 présente à cet effet une forme parallélépipédique creuse, avec une section horizontale de dimensions extérieures égales aux dimensions intérieures du pied 202, ce qui lui permet de coulisser dans le pied 202.

Cette coulisse 205 présente une extrémité inférieure ouverte vers l'intérieur du pied 202, et une extrémité supérieure fermée par une paroi plane.

La coulisse 205 présente, sur deux côtés opposés, deux ergots 204 en saillie engagés dans deux trous oblongs verticaux 203 pratiqués dans deux côtés opposés du pied 202, pour permettre le guidage de la coulisse 205 en translation suivant l'axe vertical A8, entre deux positions de butée haute et basse.

Il est par ailleurs prévu des moyens d'actionnement motorisés (non représentés) permettant de monter ou descendre la coulisse 205 dans le pied 202, à la hauteur souhaitée.

Il est également prévu des moyens de détermination (non représentés) de la hauteur de la coulisse 205 dans le pied 202 (par exemple une roue codeuse associée à une crémaillère).

Le châssis 200 présente par ailleurs deux ailes latérales 206, 207 qui bordent sa face avant tournée vers le porteur 1.

Ces deux ailes latérales 206, 207 sont formées par des parois verticales légèrement incurvées vers l'avant et sont montées sur charnière sur les deux côtés opposés du pied 202 où sont pratiqués les trous oblongs verticaux 203.

Les faces avant de ces ailes 206, 207 sont par ailleurs équipées de crochets (non représentés) sur lesquels reposent les montures de lunettes parmi lesquelles le porteur fait son choix.

Sur la figure 4, on observe que la coulisse 205 porte au moins deux (ici trois) sources de lumière 220, 230, 240 infrarouges adaptées à éclairer la tête 10 du porteur 1 en position de mesure.

Ces sources de lumière 220, 230, 240 sont préférentiellement réparties de part et d'autre du capteur d'image, dans le plan vertical du capteur d'images 210.

Ces trois sources de lumière 220, 230, 240 sont ici montées fixes en translation sur la coulisse 205 et sont formées d'une pluralité de diodes électroluminescentes (LED).

L'une des sources de lumière, appelée source principale 220, est située à faible distance du capteur d'images 210 (c'est-à-dire ici à moins de 10 centimètres du capteur d'images). Elle est composée d'une pluralité de LED réparties sur un cercle. Ces LED sont directement fixées à la coulisse 205. Telle que représentée sur la figure 4, cette source principale 220 est située au-dessus du capteur d'images 210.

Les deux autres sources de lumière, appelées sources secondaires 230, 240, sont situées l'une au-dessus de l'autre, en dessous du capteur d'images 210. Elles sont chacune composées d'un nombre de LED égal à celui de la source principale 220, réparties sur deux cercles concentriques. Elles émettent une lumière d'intensité inférieure à celle de la lumière émise par la source principale 220. Les intensités lumineuses émises par les sources secondaires 230, 240 sont, au même titre que celle émise par la source principale 220, réglables.

Ces deux sources secondaires 230, 240 sont fixées sur des socles 231, 241 montés mobiles en rotation sur la coulisse 205 autour de deux axes de rotation A6, A7 horizontaux distincts. Grâce à cette mobilité, il est possible de diriger manuellement ces sources secondaires 230, 240 vers la tête 10 du porteur 1.

Les trois sources de lumière 220, 230, 240 sont plus précisément conçues pour former des reflets distincts sur les lentilles de présentation 150G, 150D de la paire de lunettes 100 portée par le porteur 1.

Ainsi, lorsque toutes les sources de lumière 220, 230, 240 se reflètent sur les deux lentilles de présentation 150G, 150D, le capteur d'images 210 peut observer six reflets-verres.

L'utilisation de trois sources de lumière 220, 230, 240 permet au capteur d'image 201 de voir au moins une partie de ces reflets, quel que soit l'angle de tangage $\delta$ de la tête du porteur 1. On comprend en effet que lorsque le porteur 1 incline la tête vers le bas, les reflets vus par le capteur d'images 210 remontent sur les lentilles de présentation 150G, 150D jusqu'à en sortir.

Les trois sources de lumière 220, 230, 240 forment par ailleurs un unique reflet sur chaque œil du porteur 1, appelé reflet cornéen.

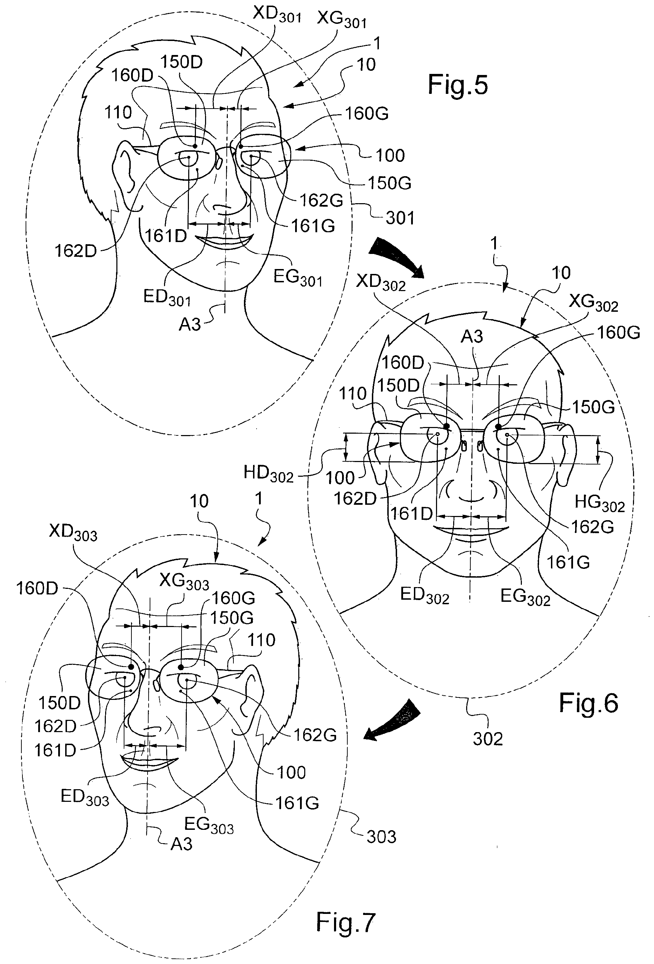

Sur la figure 6, on a représenté une situation dans laquelle seule deux des trois sources de lumière 220, 230, 240 se reflètent sur les deux lentilles de présentation 150G, 150D. On observe ainsi quatre reflets-verres 160G, 160D, 161G, 161D générés par la source principale 220 et l'une des sources secondaires 230 sur les deux lentilles de présentation 150G, 150D, et deux reflets cornéens 162G, 162D. La troisième source de lumière 240 est en revanche trop basse, eu égard à l'angle de tangage $\delta$ de la tête du porteur 1, pour générer des reflets sur les lentilles de présentation 150G, 150D.

Bien entendu, en variante, on pourrait prévoir que le kiosque ne comporte qu'une seule et unique source de lumière infrarouge.

Cette dernière pourrait être montée fixe à une position prédéterminée par rapport au capteur d'images. Il sera alors préférable d'utiliser une source de lumière de taille assez importante pour générer un reflet cornéen détectable. Cette source de lumière devra par ailleurs être placée de telle sorte que les deux reflets-verres qu'elle génère soient sensiblement situés à mi-hauteurs des lentilles de présentation 150G, 150D (c'est-à-dire sur l'axe passant par les centres des deux cadres boxing de la monture) lorsque la tête 10 du porteur 1 est idéalement placée (plan de Francfort P3 confondu avec le plan horizontal du capteur d'images 210 et plan sagittal P2 confondu avec le plan vertical du capteur d'images 210), compte tenu de l'angle pantoscopique moyen des montures.

Il conviendra alors de vérifier pendant les mesures que l'angle de tangage de la tête du porteur reste réduit, de sorte que le capteur d'image puisse voir les reflets de cette source de lumière sur les lentilles de présentation.

Selon une autre variante, on pourra prévoir que cette unique source de lumière soit montée mobile en translation verticale par rapport au capteur d'images, pour compenser l'angle de tangage de la tête du porteur.

Tel que le montre la figure 4, le kiosque 200 comporte ici un seul et unique capteur d'images 210, d'axe optique A2 horizontal.

Ce capteur d'image est ici formé par une caméra 210 adaptée à acquérir des images dans le proche infra-rouge et dans le visible.

Cette caméra présente ici les références suivantes : Sony® FCB-EX490. Elle est équipée d'un filtre infrarouge basculant entre une position normale dans laquelle ce dernier filtre les infrarouges pour que la caméra 210 puisse acquérir une image dans le domaine du visible (appelée dans la suite « image-visible »), et une position escamotée dans laquelle il laisse les infrarouges passer jusqu'au capteur qui peut alors acquérir une image dans le domaine infrarouge (appelée dans la suite « image-infrarouge »).

Cette caméra est ainsi adaptée à acquérir des images-infrarouges du porteur sur lesquelles apparaissent nettement les reflets-verres et les reflets cornéens, ainsi que des images-visibles du porteur permettant à ce dernier de vérifier que la paire de lunettes 100 sélectionnée lui sied bien.

Bien entendu, en variante, il sera possible de prévoir non pas une seule caméra, mais deux caméras distinctes, l'une adaptée à acquérir des images-infrarouges et l'autre adaptée à acquérir des images-visibles.

Ici, la paroi avant de la coulisse 205 présente une fenêtre fermée par un miroir sans tain, à l'arrière duquel se trouve la caméra 210. La caméra 210 est ainsi invisible depuis l'extérieur du kiosque 200, mais elle demeure adaptée à acquérir des images des individus placés à l'avant du kiosque 200.

La caméra 210 est alors installée dans le kiosque 200 de telle manière que son objectif soit situé au contact ou à proximité de la face arrière de ce miroir sans tain.

L'objectif de la caméra 210 est par ailleurs entouré d'une paroi latérale opaque, permettant d'éviter que des reflets parasites n'apparaissent sur les images acquises.

En l'espèce, l'ensemble de la caméra est ici logé dans une boîte opaque qui est ouverte vers l'avant par une ouverture au travers de laquelle émerge l'objectif, cette boîte étant située au contact de la face arrière du miroir sans tain.

Le kiosque à lunettes 200 comporte par ailleurs au moins une cible 310 située sur le côté et à distance réduite de l'objectif de la caméra 210, de telle sorte qu'elle est visible par le porteur 1 en position de mesure.

Cette cible 310 comporte ici une fenêtre 208 pratiquée dans la paroi de la coulisse 205, entre l'objectif de la caméra 210 et les sources secondaires 230, 240, au travers de laquelle une LED est visible. En pratique, cette LED est ici située dans le pied 202 du châssis 200, et est réfléchie vers la fenêtre 208 au moyen d'un jeu de miroir.

Cette cible 310 permet d'attirer le regard du porteur 1 vers l'objectif de la caméra 210 lors des mesures.

Il est également prévu deux cibles supplémentaires 320, 330, situées sur les bords extérieurs de chacune des ailes 206, 207 du châssis 200, dans le plan horizontal de la caméra 210.

Comme cela sera bien exposé dans la suite de ce texte, ces cibles supplémentaires 320, 330 permettront d'attirer séquentiellement le regard du porteur 1 vers l'un des côtés du kiosque 200 puis l'autre, afin de déterminer si ce porteur 1 présente une propension à détourner le regard en bougeant plutôt la tête 10 ou les yeux 20G, 20D.

Il est par ailleurs prévu deux néons 260, respectivement positionnés sur les bords supérieurs des deux ailes 206, 207 du châssis 200, qui éclairent la tête 10 du porteur 1 de telle sorte que les images-visibles acquises par la caméra 210 soient bien exposées.

Il est aussi prévu dans l'une de ces deux ailes 206 des moyens d'affichage 270 et des moyens d'impression 280 des images-visibles acquises.

Les moyens d'affichage sont ici constitués par un écran tactile 270 fixé sur la face avant de l'aile 206, de manière à être visible par le porteur 1 en position de mesure.

Les moyens d'impression sont quant à eux conçus pour imprimer des fiches récapitulatives des données de commande des lentilles ophtalmiques, sur lesquelles apparaissent les photos des porteurs.

Il est par ailleurs prévu des moyens pour scanner les prescriptions du porteur 1.

Ici, les moyens d'impression et les moyens pour scanner sont confondus et sont constitués par une unique imprimante multifonction 280 couleur, située dans un logement prévu en creux dans la face avant de l'aile 206 du châssis 200.

Bien entendu, en variante, il serait également possible de prévoir deux appareils distincts, l'un pour imprimer les fiches récapitulatives et l'autre pour scanner les prescriptions.

Il est enfin prévu sur l'autre des deux ailes 207 un moyen d'acquisition d'au moins une dimension de la paire de lunettes 100. Les images acquises par la caméra 210 ne permettent en effet pas de déterminer les dimensions de la paire de lunettes 100 du porteur 1. Il est alors nécessaire de mettre à l'échelle les images acquises.

Les moyens d'acquisition 290 précités peuvent alors se présenter sous diverses formes.

De manière préférentielle, ils comportent un lecteur de code-barres 290 connecté à une base de données dont chaque enregistrement est associé à une référence de montures de lunettes et comprend un identifiant et des données relatives à cette référence de montures de lunettes.

En l'espèce, l'identifiant de chaque enregistrement est formé par le numéro du code-barres qui est assigné à la référence de montures de lunettes.

Les données mémorisées dans chaque enregistrement comportent quant à elles ici :

- la largeur totale des montures de lunettes de la référence considérée, mesurée entre les deux branches 112D, 112G,
- le rapport entre la hauteur et la largeur des cadres boxing 151G, 151D des cercles de ces montures de lunettes, et
- l'écart entre ces deux cadres boxing 151G, 151D.

Bien entendu, les données mémorisées dans chaque enregistrement de la base de donnée pourraient comporter un nombre réduit d'éléments (par exemple seulement la largeur totale de la monture de lunettes) ou un nombre accru d'éléments (par exemple la forme exacte des cercles, le matériau des montures de lunettes, l'angle pantoscopique et l'angle de galbe des montures de lunettes, ...).

En variante, ces moyens d'acquisition pourraient ne comporter qu'un simple clavier (physique ou affiché sur l'écran tactile) permettant au vendeur :

- de saisir la largeur de la monture de lunettes 110 sélectionnée, qu'il aura au préalable mesurée à l'aide d'un réglet, et
- de positionner sur l'image acquise deux curseurs au niveau des 2 points entre lesquels il aura mesuré la largeur de la monture.

Encore en variante, ces moyens d'acquisition pourraient ne comporter qu'un simple étalon de dimensions connues, à rapporter sur la monture de lunettes sélectionnée (par clipsage, collage ou tout autre moyen) pour obtenir sur chaque image acquise par la caméra une référence dont les dimensions sont connues, permettant ainsi de mettre cette image à l'échelle.

Comme le montre la figure 4, l'unité de calcul 250 du kiosque à lunettes 200 est quant à elle logée à l'intérieur du pied 202 du châssis 200. Elle est conçue pour piloter les différents composants électroniques du kiosque 200 et pour traiter les images acquises par la caméra 210.

Cette unité de calcul 250 comprend à cet effet un processeur (CPU), une mémoire vive (RAM), une mémoire morte (ROM), des convertisseurs analogiques-numériques (A/D), et différentes interfaces d'entrée, de sortie et de communication.

Grâce à ses interfaces d'entrée, l'unité de calcul 250 est adaptée à recevoir les images acquises par la caméra 210, la hauteur de la coulisse 205 dans le pied 202 mesurée par lesdits moyens de détermination, et le numéro de code-barres de la monture de lunettes sélectionnée, lu par le lecteur de code-barres 290.

Dans sa mémoire vive, l'unité de calcul 250 mémorise ainsi en continu ces différentes données.

Grâce à un logiciel enregistré dans sa mémoire morte, l'unité de calcul 250 est adaptée à mettre en œuvre l'ensemble du procédé qui sera décrit dans la suite de cet exposé. Elle est ainsi par exemple adaptée à générer des signaux de pilotage des moyens d'actionnement de la bascule 205 pour positionner cette dernière à la hauteur souhaitée, et des signaux de communication comportant les données de commande des lentilles ophtalmiques.

Grâce à ses interfaces de sortie, l'unité de calcul 250 est adaptée à transmettre ces signaux de sortie aux différents composants électroniques du kiosque 200, notamment à la caméra 210, aux sources de lumière 220, 230, 240, à l'écran tactile 270, à l'imprimante multifonction 280, aux cibles 310, 320, 330 et aux moyens d'actionnement de la coulisse 205.

Grâce à ses interfaces de communication, l'unité de calcul 250 est adaptée à transmettre les signaux de communication à un centre de fabrication de lentilles ophtalmiques.

Il est enfin prévu un interrupteur de mise sous tension du kiosque 200.

Préalablement à la venue du porteur 1, le vendeur met le kiosque 200 sous tension à l'aide de l'interrupteur prévu à cet effet.

Lors de cette mise sous tension, l'unité de pilotage commande l'alimentation électrique des néons 260, de manière à mettre en lumière les paires de lunettes qui sont à disposition sur les ailes 206, 207 du châssis 201 du kiosque 200.

Les trois sources de lumière 220, 230, 240 infrarouges restent quant à elles éteintes.

Puis, lorsqu'un individu se présente, après lecture d'un message sur l'écran l'y invitant, il choisit une paire de lunettes 100 parmi l'ensemble de celles qui sont en présentation sur les ailes 206, 207 du châssis 201 du kiosque 200. Dans l'exemple représenté sur les figures, la paire de lunettes sélectionnée est du type cerclé. Puis, comme l'y invite un message affiché sur l'écran, l'individu appelle le vendeur pour la suite du protocole.

Il est ensuite prévu une opération de détermination d'une dimension caractéristique de la monture de lunettes 110 sélectionnée.

Comme cela a été exposé supra, cette opération est notamment prévue pour permettre de mettre les images acquises à l'échelle.

Au cours de cette opération, le vendeur passe le code-barres de la monture de lunettes 110 sélectionnée devant le lecteur de code-barres 290. L'unité de calcul 250, grâce au numéro de code-barres, cherche alors dans la base de données l'enregistrement correspondant à cette monture de lunettes 110, puis elle récupère et mémorise dans sa mémoire morte les données suivantes : la largeur totale de la monture de lunettes 110, le rapport entre la hauteur et la largeur des cadres boxing 151G, 151D des cercles 111D, 111G de cette monture de lunettes 110, et l'écart entre ces deux cadres boxing 151G, 151D.

Bien entendu, comme cela a été exposé supra, cette opération de lecture de code-barres pourrait être remplacée par une opération plus simple consistant pour le vendeur à mesurer la largeur totale de la monture de lunettes à l'aide d'un réglet, à la saisir sur un clavier virtuel affiché sur l'écran tactile, et à positionner sur l'image acquise les deux curseurs au niveau des deux points entre lesquels il a mesuré la largeur de la monture (cette opération de positionnement des deux points pouvant en variante être réalisée automatiquement).

Il est alors prévu une opération d'acquisition des prescriptions du porteur 1, que ce dernier aura préalablement obtenues chez un optométriste.

Ces prescriptions sont généralement inscrites sur une feuille de prescription et comportent en particulier le type de lentilles (simples, bifocales, à variation progressive de puissances, teintées, ...) et le pouvoir de réfringence que devront présenter les lentilles pour corriger les déficiences de vision du porteur (c'est-à-dire leurs puissances optiques sphériques, cylindriques et prismatiques et leurs axes de cylindre). Elles peuvent bien sûr comporter d'autres informations telles que, dans le cas des lentilles bifocales ou à variation progressive de puissances, une addition.

Lors de cette opération, le vendeur recueille alors la feuille de prescription et la scanne à l'aide de l'imprimante multifonction 280, de manière que l'unité de calcul 250 puisse mémoriser l'image scannée de cette feuille de prescription dans sa mémoire morte.

Le vendeur peut également demander au porteur de choisir les traitements qu'il souhaite obtenir sur ses lentilles ophtalmiques (anti-reflets, hydrophobes, ...) et saisir ces informations dans des champs affichés à cet effet par l'unité de calcul 250 sur l'écran tactile 270.

Une fois ces informations acquises, l'unité de calcul 250 commande la mise sous tension de la première cible 310.

Elle commande également l'affichage sur l'écran tactile 270 :

- d'un message indiquant que les opérations de mesure peuvent commencer,
- des images acquises « en temps réel » par la caméra 210, et
- de deux flèches orientées à l'opposée l'une de l'autre, l'une vers le haut et l'autre vers le bas, pour piloter le déplacement de la coulisse 205 vers le haut ou vers le bas.

Le vendeur invite alors tout d'abord le porteur 1 à chausser ses lunettes et à les garder ainsi pendant l'ensemble des examens qui vont suivre.

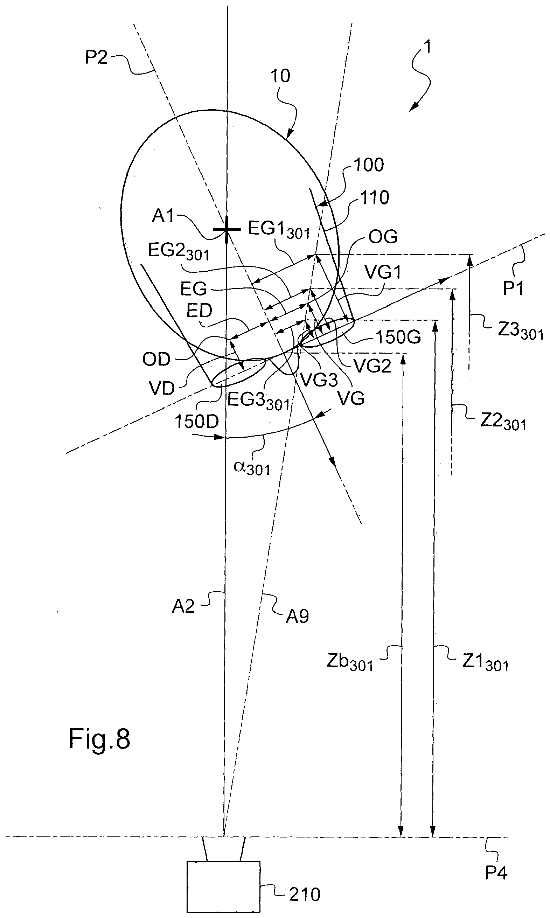

Comme cela sera exposé en détail dans la suite de cette description, ces examens permettront alors au kiosque 200 de déterminer les demi-écarts pupillaires EG, ED et les hauteurs pupillaires HG, HD du porteur 1, ainsi que des paramètres de personnalisation avancée tels que la distance VG, VD entre la monture de lunettes 110 et chaque œil du porteur 1 (figure 8), le comportement en mobilité de son regard...

Il demande pour cela au porteur 1 de se placer face au kiosque 200, en position de mesure, et de regarder la première cible 310 en gardant la tête droite, de telle manière que son plan de Francfort P3 soit sensiblement horizontal et que son plan sagittal P2 soit sensiblement vertical.

Ici, le porteur 1 est invité à se placer debout, face à la coulisse 205 du châssis 201 du kiosque 200.

Le vendeur règle alors la position de la coulisse 205 à une hauteur adaptée à la taille du porteur 1. Il utilise à cet effet les flèches affichées sur l'écran tactile 270 pour commander la montée ou la descente de la coulisse 205 jusqu'à une hauteur telle que l'ensemble du visage du porteur 1 apparaisse sur les images acquises par la caméra 210 et affichées sur l'écran tactile 270.

Ainsi, dans cette position, le visage du porteur 1 se trouve tout entier dans le champ de la caméra 210.

Cette opération de positionnement de la caméra 210 à la hauteur de la tête 10 du porteur 1 pourrait bien entendu être réalisée autrement.

Elle pourrait ainsi être réalisée automatiquement par l'unité de calcul qui traiterait les images acquises par la caméra de manière à y repérer la monture de lunettes et qui piloterait en conséquence la bascule à une hauteur telle que la monture de lunettes se trouve au centre des images acquises par la caméra.

En variante, si le capteur d'images n'est pas monté mobile par rapport au sol, cette opération de positionnement pourra être réalisée en demandant au porteur de s'asseoir sur un tabouret dont la hauteur aura au préalable été réglée.

Une fois cette opération de positionnement du porteur 1 par rapport à la caméra 210 réalisée, le vendeur initie la prise de mesure en appuyant sur un bouton ad hoc affiché sur l'écran tactile 270.

L'unité de pilotage 250 commande alors l'allumage des trois sources de lumière 220, 230, 240 infrarouges, à une intensité nominale.

Bien entendu, l'unité de pilotage pourrait moduler cette intensité, en fonction de l'intensité de la lumière extérieure, pour éviter que cette dernière ne perturbe les mesures. L'utilisation d'une lumière infrarouge permet toutefois de limiter ces perturbations, si bien que l'intensité nominale est généralement suffisante.

L'utilisation de trois sources de lumière 220, 230, 240 distinctes assure alors la présence d'au moins un reflet sur chaque lentille de présentation 150G, 150D de la paire de lunettes 100, pour autant bien sûr que le porteur 1 ne détourne pas complètement la tête.

Dans la variante où le kiosque ne comporterait qu'une source de lumière mobile en hauteur par rapport à la caméra, l'opération de positionnement du porteur en face de la caméra serait suivie d'une opération automatique de positionnement de la source de lumière. Au cours de cette opération, l'unité de calcul ferait varier la hauteur de la source de lumière et traiterait les images acquises par la caméra pour immobiliser la source de lumière à une hauteur telle que les reflets-verres soient centrés sur les lentilles de présentation.

Quoi qu'il en soit, le vendeur demande alors au porteur 1 de tourner la tête 10 de droite à gauche et de gauche à droite, autour de l'axe longitudinal A1 (de manière à faire « non » de la tête).

Il lui indique que le mouvement doit être réalisé lentement (avec une période supérieure à la seconde), avec une amplitude réduite (d'environ 10 degrés).

En variante, on pourrait prévoir de monter la caméra et les sources de lumière mobiles de manière à ce qu'elles pivotent ensemble autour de l'axe longitudinal de la tête du porteur. Ainsi, le porteur n'aurait pas à faire de mouvement de tête.

Puis, lorsque le porteur 1 commence à tourner la tête de la manière demandée, le vendeur appuie sur un bouton de démarrage des mesures affiché sur l'écran tactile 270.

La caméra acquiert alors une pluralité d'images-infrarouges, sur lesquelles apparaissent la tête 10 du porteur 1 et sa paire de lunettes 100.

Les images acquises sont tout d'abord mémorisées dans la mémoire vive de l'unité de calcul 250. Certaines d'entre elles, jugées exploitables, sont alors sélectionnées par l'unité de calcul 250 et mémorisées dans sa mémoire morte. La sélection des images acquises est réalisée de la manière suivante.

Une image acquise peut présenter plusieurs reflets-verres (au maximum 6), chaque reflet-verre 160G, 160D, 161G, 161D est noté individuellement sur différents critères, notamment :

- de forme (largeur, hauteur, ratio largeur/hauteur), et
- d'intensité (luminance).

Chaque couple de reflets (générés par une même source de lumière et respectivement réfléchis par les deux lentilles de présentation) est par ailleurs noté selon d'autres critères, notamment :

- de distance entre les reflets,
- d'horizontalité par rapport à l'axe horizontal des cadres boxing, et
- de comparaison des surfaces des reflets.

Ici, la note attribuée à chaque critère n'est pas binaire.

On peut par exemple prévoir qu'elle varie continûment entre 0 et 1 de telle sorte que :

- elle soit égale à 1 si le critère est compris entre 90% et 110% de sa valeur nominale (prédéterminée et mémorisée dans la mémoire morte de l'unité de calcul 250),
- elle soit égale à 0 si le critère est supérieur à 120% ou inférieur à 70% de sa valeur nominale, et
- elle varie linéairement entre 70% et 90% et entre 110% et 120%.

Les critères s'appliquant aux reflets-verres peuvent également s'appliquer mutatis mutandis aux reflets cornéens 162G, 162D. Un critère supplémentaire s'appliquant aux reflets cornéens sera la distance séparant chaque reflet cornéen de l'axe remarquable A3.

Alors, une image a d'autant plus de chance d'être sélectionnée que le produit des notes attribuées aux différents critères précités est important.

On peut par exemple prévoir que les images sélectionnées sont celles pour lesquelles le produit des notes est supérieur à un seuil prédéterminé.

En variante, on peut prévoir que les images sélectionnées sont celles pour lesquelles les produits des notes sont les plus élevés.

On peut par ailleurs prévoir qu'une image est automatiquement rejetée si l'une des notes qui lui a été attribuée est inférieure à un autre seuil prédéterminé.

Ainsi, si sur une image acquise, le porteur 1 a tourné la tête d'un angle de lacet $\alpha$ trop important et que les reflets-verres sont sortis du contour des lentilles de présentation 150G, 150D, cette image est automatiquement rejetée.

Par ailleurs, si sur une image acquise, le porteur 1 a incliné la tête d'un angle de roulis $\beta$ important et que les reflets-verres générés par une même source de lumière son décalés en hauteur sur les deux lentilles de présentation, cette image pourra être rejetée si ce décalage est vraiment très important, ou sélectionnée si les notes attribuées aux autres critères sont élevées.

L'unité de calcul 250 obtient ainsi un nombre restreint d'images-infrarouges exploitables pour la suite du procédé.

Pour la clarté de cet exposé, on considérera alors que l'unité de calcul 250 n'a sélectionné que trois images-infrarouges 301, 302, 303 représentées sur les figures 5 à 7.

Préférentiellement, l'unité de calcul arrêtera l'acquisition d'images lorsqu'elle aura sélectionnée au moins 10 images différentes.

L'unité de calcul 250 commande ensuite le basculement du filtre infrarouge de la caméra 210 pour mémoriser une image-visible du visage du porteur 1.

Elle commande simultanément l'extinction des sources de lumières 220, 230, 240 infrarouges, et l'affichage d'un message sur l'écran tactile 270 indiquant au porteur qu'il peut arrêter de tourner la tête.

Ce message est donc affiché après que l'unité de calcul 250 a sélectionné un nombre prédéterminé d'images, ici égal à 10.

En variante, on pourrait prévoir d'afficher ce message après une durée prédéterminée, par exemple égale à 10 secondes, à l'issue de laquelle on estime pouvoir obtenir un nombre suffisant d'images exploitables. Les images pourront alors être sélectionnées dans un second temps, après que l'ensemble des images ont été acquises.

Une fois les images 301, 302, 303 sélectionnées, l'unité de calcul 250 procède au calcul de l'angle de lacet $\alpha$ de la tête 10 du porteur 1 sur chacune de ces images.

Le calcul de cet angle de lacet $\alpha$ est opéré en localisant sur chaque image 301, 302, 303 les reflets-verres 160D, 160G, 161D, 161G par rapport au plan sagittal P2 de la tête du porteur 1.

Ici, cet angle de lacet $\alpha$ est plus précisément déterminé en localisant les reflets-verres 160D, 160G, 161D, 161G par rapport à l'axe remarquable A3 de la monture de lunettes (dont la position correspond en effet sensiblement à l'intersection du plan sagittal P2 avec le plan moyen P1 de la monture de lunettes 110).

La position de l'axe remarquable A3 de la monture de lunettes 110 est obtenu en 3 étapes consistant à :

- repérer sur chaque image 301, 302, 303 la monture de lunettes 110, et notamment les cercles 111D, 111G de cette monture de lunettes,
- positionner sur chaque image 301, 302, 303 les cadres boxing 151G, 151D, et
- déterminer la position de l'axe passant entre ces deux cadres boxing, qui correspond en pratique à l'axe remarquable A3.

L'utilisation d'une lumière infrarouge permet ici de faciliter le repérage de la monture de lunettes 110, dont le contour se détache bien de celui du visage du porteur 1.

Alors, l'angle de lacet $\alpha$ de la tête 10 du porteur 1 sur chaque image 301, 302, 303 est calculé en fonction des distances séparant les reflets-verres 160D, 160G, 161D, 161G de l'axe remarquable A3.

Typiquement, comme le montrent bien les figures 5 à 7, l'unité de calcul 250 détermine sur chaque image 301, 302, 303 les distances $XD_{301}$, $XG_{301}$, $XD_{302}$, $XG_{302}$, $XD_{303}$, $XG_{303}$ séparant l'axe remarquable A3 des centres des deux reflets-verres 160G, 160D les plus gros (les reflets-verres générés par les sources secondaires n'étant exploités que si les reflets-verres générés par la source principale n'apparaissent par sur les images acquises).

L'angle de lacet $a_i$ de la tête 10 du porteur 1 sur chaque image 301, 302, 303 est alors déterminé de la manière suivante :

$$\alpha_i = k. \ (XG_i - XD_i) \ / \ (XG_i + XD_i),$$

avec i allant de 301 à 303.

Le coefficient k est alors une constante relative au galbe de la monture de lunettes.

Ce coefficient k pourra être formé par une constante prédéterminée et invariable, mémorisée dans la mémoire morte de l'unité de calcul 250. Elle sera donc toujours la même quelle que soit la monture de lunettes sélectionnée par le porteur. Ce coefficient k pourra alors être déterminé expérimentalement sur la base d'un panel représentatif de montures de lunettes, et ainsi choisi environ égal à 40°.

En variante, ce coefficient k pourra être formé par une constante prédéterminée associée à la monture de lunettes sélectionnée par le client. Dans cette variante, ce coefficient k sera alors déterminé expérimentalement pour chaque référence de monture de lunettes, puis mémorisé dans la base de données de manière à être accessible lors du calcul de l'angle de lacet $a_i$ de la tête 10 du porteur 1.

On pourrait par ailleurs prévoir que le calcul de cet angle de lacet soit affiné ou vérifié, en procédant à un second calcul de cet angle de lacet qui ne tienne pas compte de la position des reflets-verres.

Ainsi, on pourrait prévoir d'équiper la monture de lunettes d'un système de repérage comportant des motifs géométriques facilement repérables et écartés d'une distance réelle connue, et de déterminer l'angle de lacet en fonction de la distance séparant ces motifs géométriques sur l'image acquise (préalablement mise à l'échelle 1 : 1). Un tel procédé est présenté en détail dans le document WO 2008/132356.

A ce stade, l'unité de calcul 250 a en mémoire les angles de lacet $\alpha_{301}$, $\alpha_{302}$, $\alpha_{303}$ de la tête 10 du porteur 1 sur chaque image 301, 302, 303 sélectionnée.

Elle procède alors à la détermination des demi-écarts pupillaires EG, ED du porteur 1, en fonction des positions des reflets cornéens 162G, 162D du porteur 1 sur au moins une des images 301, 302, 303 sélectionnées.

Elle peut pour cela procéder de différentes manières. Deux méthodes différentes seront ici exposées.

La première méthode consiste à élire une image 302 parmi les images 301, 302, 303 sélectionnées, à savoir celle pour laquelle l'angle de lacet $\alpha_{302}$ calculé est le plus faible, puis à considérer que cette image élue 302, la tête 10 du porteur 1 est de face. Les demi-écarts pupillaires EG, ED pourront alors être directement lus sur cette image 302.

Cette méthode nécessite toutefois d'avoir acquis et sélectionné un nombre suffisant d'images 301, 302, 303, de manière que parmi ces images, l'une d'entre elles représente effectivement la tête du porteur 1 vue sensiblement de face.

Cette méthode est alors plus précisément mise en oeuvre de la manière suivante.

L'unité de calcul 250 procède au repérage sur cette image élue 302 des reflets cornéens 162G, 162D, qui sont excentrés par rapport aux reflets-verres.

Puis, l'unité de calcul 258 détermine les distances $EG_{302}$, $ED_{302}$ séparant l'axe remarquable A3 de chacun de ces reflets cornéens 162G, 162D.

Ces distances $EG_{302}$, $ED_{302}$ sont ensuite mises à l'échelle, ce qui permet à l'unité de calcul 250 d'obtenir les demi-écarts pupillaires EG, ED du porteur 1.

La seconde méthode consiste à considérer toutes les images 301, 302, 303 sélectionnées, puis, par une méthode statistique, à évaluer les demi-écarts pupillaires EG, ED du porteur 1.

Cette méthode statistique permet non seulement d'évaluer les demi-écarts pupillaires EG, ED du porteur 1, mais également d'évaluer les distances œil-lunettes VG, VD séparant le plan moyen P1 de la monture de lunettes 110 et les centres de rotation OG, OD des yeux 20G, 20D du porteur 1.

Cette méthode statistique est alors mise en œuvre sur l'un des deux yeux 20G, 20D du porteur 1.

Ici, comme le montre la figure 8, elle sera mise en œuvre sur l'œil gauche 20G du porteur 1 afin de déterminer son demi-écart pupillaire gauche EG et sa distance œil-lunettes gauche VG.

Globalement la méthode statistique part du constat que, grâce au reflet cornéen gauche 162G apparaissant sur chaque image 301, 302, 303, on sait sur quel axe A9 (voir figure 8) se trouve le centre de rotation OG de l'œil gauche 20G du porteur 1, et qu'il reste donc seulement à déterminer la position du centre de rotation OG sur cet axe A9.

Cette méthode statistique va alors consister :

- à simuler, sur chaque image 301, 302, 303, une pluralité de distances œil-lunettes VG1, VG2, VG3 (ici au nombre de 3 pour la clarté de l'exposé et des dessins),
- à déterminer le demi-écart pupillaire gauche correspondant à chacune de ces distances simulées (appelé « demi-écart pupillaire gauche simulé $EG1_{301}$, $EG2_{301}$, $EG3_{301}$, $EG1_{302}$, $EG2_{302}$, $EG3_{302}$, $EG1_{303}$, $EG2_{303}$, $EG3_{303}$ »), puis, en fonction de l'ensemble de ces demi-écarts pupillaires gauches simulés,
- à déterminer laquelle des distances œil-lunettes simulées VG1, VG2, VG3 est la plus proche de la distance œil-lunette VG réelle du porteur 1.

Plus précisément, l'unité de calcul 250 considère ici trois distances œil-lunettes simulées VG1, VG2, VG3 prédéterminées et mémorisées dans sa mémoire morte. En variante et de manière préférentielle, elle considérera plutôt un nombre de distances œil-lunettes simulées supérieur à 10.

**EP 2 822 451 B1**

Alors, pour chacune de ces distances œil lunettes simulées, et pour chacune des images acquises (indice i, variant dans cet exemple de 301 à 303), l'unité de calcul 250 calcule de manière itérative (indice d'itération k variant de 1 à n) :

$$Z1_{i,k} = Zb_i + VG1.\cos(\alpha_i) + EG1_{i,k-1}.\sin(\alpha_i),$$

$$Z2_{i,k} = Zb_i + VG2.\cos(\alpha_i) + EG2_{i,k-1}.\sin(\alpha_i),$$

$$Z3_{i,k} = Zb_i + VG3.\cos(\alpha_i) + EG3_{i,k-1}.\sin(\alpha_i),$$

avec :

- $EG1_{i,0}$, $EG2_{i,0}$, $EG3_{i,0}$ fixé arbitrairement à une valeur prédéterminée, ici choisie égale à 32,5 mm ;
- $Zb_i$ la distance séparant l'axe remarquable A3 et le plan général P4 de l'objectif de la caméra 210 sur chaque image (et qui est facilement déterminable, compte tenu de la largeur de la monture sur l'image considérée par rapport à sa largeur réelle, et de l'angle de lacet $\alpha_i$ de la tête du porteur) ;
- $Z1_{i,k}$, $Z2_{i,k}$, $Z3_{i,k}$ les distances séparant la position simulée du centre de rotation OG de l'œil gauche 20G du porteur 1 et le plan général P4 de l'objectif de la caméra 210.

Si on note :

- $x_{OGi}$ et $y_{OGi}$ : les coordonnées, exprimées en pixel, du centre de rotation OG dans l'image i, par rapport au centre de cette image,
- X,Y,Z : les coordonnées, exprimées en mm, du centre de rotation OG dans le repère de la caméra, et
- K, la taille d'un pixel, exprimée en mm, pour un objet situé à un mètre du plan de la caméra.

Alors, on peut écrire les équations suivantes :

$$X1_{i,k} = Z1_{i,k} . K . x_{OGi}$$

$$Y1_{i,k} = Z1_{i,k} . K . y_{OGi}$$

$$Z1_{i,k} = Z1_{i,k}$$

$$X2_{i,k} = Z2_{i,k} . K . x_{OGi}$$

$$Y2_{i,k} = Z2_{i,k} . K . y_{OGi}$$

$$Z2_{i,k} = Z2_{i,k}$$

$$X3_{i,k} = Z3_{i,k} . K . x_{OGi}$$

$$Y3_{i,k} = Z3_{i,k} . K . y_{OGi}$$

$$Z3_{i,k} = Z3_{i,k}$$

13

Pour l'image i, le plan sagittal, noté $P2_i$ peut être, par exemple, défini comme le plan qui inclut les trois points suivants :

- $A_i$ : un point situé sur l'axe remarquable A3, correspondant au centre du segment reliant les deux coins supérieurs les plus proches des deux cadres boxing 151G, 151D,
- $B_i$ : un point situé sur l'axe remarquable A3, correspondant au centre du segment reliant les deux coins inférieurs les plus proches des deux cadres boxing 151G, 151D,
- $C_i$: un point situé sur un axe qui passe par le point $A_i$, qui est perpendiculaire au plan moyen P1 de la monture de lunettes 110, et dont les coordonnées dans le repère de la monture sont telles que le vecteur $A_iC_i$ a pour coordonnées $(x_c, y_c, z_c)$ avec $x_c = 0$ et $y_c = 0$.

Si on pose :

- $(x_{Ai}, y_{Ai})$ les coordonnées du point $A_i$ dans l'image i,
- $X_{Ai}, Y_{Ai}, Z_{Ai}$, les coordonnées du point $A_i$ dans le repère de la caméra,
- $(x_{Bi}, y_{Bi})$ les coordonnées du point $A_i$ dans l'image i,
- $X_{Bi}, Y_{Bi}, Z_{Bi}$ les coordonnées du point $B_i$ dans le repère de la caméra,
- $X_{Ci}, Y_{Ci}, Z_{Ci}$ les coordonnées du point $C_i$ dans le repère de la caméra, et
- $M_i$, la matrice de rotation tridimensionnelle décrivant la posture de la monture dans l'espace de la caméra (construite à partir des angles $\alpha$, $\beta$ et $\delta$).

Alors, comme la distance de l'axe remarquable A3 au plan de la caméra P4 est connue, $Z_{Ai}$ et $Z_{Bi}$ sont connus et on peut écrire les équations suivantes :

$$X_{Ai} = Z_{Ai} \, . \, K \, . \, x_{Ai}$$

$$Y_{Ai} = Y_{Ai} \, . \, K \, . \, y_{Ai}$$

$$X_{Bi} = Z_{Bi} \, . \, K \, . \, x_{Bi}$$

$$Y_{Bi} = Y_{Bi} \, . \, K \, . \, y_{Bi}$$

Et, après inversion de la matrice de rotation M :

$$(X_{Ci}-X_{Ai}) = M^{-1}(0,0) \, . \, x_c + M^{-1}(0,1) \, . \, y_c + M^{-1}(0,2) \, . \, z_c$$

$$(Y_{Ci}-Y_{Ai}) = M^{-1}(1,0) \, . \, x_c + M^{-1}(1,1) \, . \, y_c + M^{-1}(1,2) \, . \, z_c$$

$$(Z_{Ci}-Z_{Ai}) = M^{-1}(2,0) \, . \, x_c + M^{-1}(2,1) \, . \, y_c + M^{-1}(2,2) \, . \, z_c$$

Comme, $x_c$ et $y_c$ sont égaux à zéro par construction, on en déduit que :

$$X_{Ci} = X_{Ai} + M^{-1}(0,2) \, . \, z_c$$

$$Y_{Ci} = Y_{Ai} + M^{-1}(1,2) \, . \, z_c$$

$$Z_{Ci} = Z_{Ai} + M^{-1}(2,2) \, . \, z_c$$

Les coordonnées des 3 points du plan $P2_i$ étant connues, celui-ci est parfaitement défini, et il est donc possible de calculer selon la méthode classique la distance d'un point quelconque au plan $P2_i$.

On peut donc en particulier calculer $EG1_{i,k}$, $EG2_{i,k}$, $EG3_{i,k}$, avec

- $EG1_{i,k}$ : la distance séparant le centre de rotation OG du plan $P2_i$, en considérant la distance œil-lunettes VG1 ;
- $EG2_{i,k}$ : la distance séparant le centre de rotation OG du plan $P2_i$, en considérant la distance œil-lunettes VG2 ;
- $EG3_{i,k}$ : la distance séparant le centre de rotation OG du plan $P2_i$, en considérant la distance œil-lunettes VG3.

Ces valeurs peuvent alors être réinjectées dans les formules initiales pour continuer l'itération.

Le calcul itératif s'arrête soit après un nombre prédéterminé d'itérations (par exemple pour k = 3), soit lorsque pour deux itérations successives, les valeurs de $EG1_{i,k}$ et $EG1_{i,k+1}$, celles de $EG2_{i,k}$ et $EG2_{i,k+1}$ et celles de $EG3_{i,k}$ et $EG3_{i,k+1}$ sont proches (à savoir ici inférieures à 0,1 mm).

Les valeurs de $EG1_{i,k}$, $EG2_{i,k}$, $EG3_{i,k}$ obtenues sont alors considérées comme étant les demi-écarts pupillaires simulés $EG1_i$, $EG2_i$, $EG3_i$.

Une fois l'ensemble des demi-écarts pupillaires simulés $EG1_i$, $EG2_i$, $EG3_i$ calculés, l'unité de calcul 250 sélectionne, parmi les distances œil-lunettes simulées VG1, VG2, VG3, celle qui est la plus proche de la valeur réelle VG.

Pour cela, l'unité de calcul 250 détermine, pour chaque distance œil-lunettes simulée VG1, VG2, VG3, l'écart-type $\sigma_1$, $\sigma_2$, $\sigma_3$ des demi-écarts pupillaires simulés $EG1_i$, $EG2_i$, $EG3_i$ associés.

A titre d'exemple, l'écart-type $\sigma_1$ associé à la première distance œil-lunettes simulée VG1 est égal à l'écart-type des demi-écarts pupillaires simulés $EG1_{303}$, $EG1_{302}$, $EG1_{303}$.

Alors, la distance œil-lunettes simulée VG2 sélectionnée est celle pour laquelle l'écart-type $\sigma_2$ est le plus faible.

L'unité de calcul 250 considère alors que cette distance œil-lunettes simulée VG2, une fois mise à l'échelle, est égale à la distance œil-lunettes VG réelle du porteur 1. Elle mémorise donc sa valeur.

L'unité de calcul 250 calcule ensuite la moyenne des demi-écarts pupillaires simulés $EG2_{301}$, $EG2_{302}$, $EG2_{303}$ associés à cette valeur simulée sélectionnée VG2.

Elle considère ensuite que cette moyenne, une fois mise à l'échelle, est égale au demi-écart pupillaire gauche EG du porteur 1. Elle mémorise donc sa valeur.

La détermination de la distance œil-lunettes droite VD et du demi-écart pupillaire droit ED du porteur 1 peut alors être réalisée de la même manière que pour les distances œil-lunettes gauche VG et demi-écart pupillaire gauche EG du porteur 1.

Au cours d'une opération suivante, l'unité de calcul 250 procède au calcul des hauteurs pupillaires HG, HD du porteur 1 (voir figure 1).

Encore une fois, l'unité de calcul 250 peut opérer de diverses manières.

Ici, on considérera que le tangage de la tête 10 du porteur 1 a peu d'incidence sur la précision des hauteurs pupillaires HG, HD que l'on peut mesurer sur les images 301, 302, 303.

Par conséquent, pour déterminer ces hauteurs pupillaires, l'unité de calcul 250 sélectionne l'une quelconque des images 301, 302, 303 (par exemple celle pour laquelle l'angle de lacet $\sigma_{302}$ est le plus faible) puis détermine sur cette image 302 (figure 6) les distances $HG_{302}$, $HD_{302}$ séparant chacun des reflets cornéens 162G, 162D du bord inférieur du cadre boxing 151G, 151D correspondant.

Ces distances $HG_{302}$, $HD_{302}$, une fois mises à l'échelle, sont alors mémorisées par l'unité de calcul 250 comme étant les hauteurs pupillaires HG, HD.

En variante, l'unité de calcul pourra mesurer ces distances $HG_i$, $HD_i$ sur chacune des images 301, 302, 303, moyenner ces distances et les mettre à l'échelle afin d'obtenir les hauteurs pupillaires du porteur.

Selon une autre variante, on pourra procéder au calcul des hauteurs pupillaires en suivant une méthode homologue de celle utilisée pour déterminer les demi-écarts pupillaires du porteur. Cette méthode consistera par exemple à acquérir différentes images du porteur faisant « oui » de la tête, à déterminer les angles de tangage de la tête sur chacune de ces images, et à mesurer les hauteurs pupillaires sur l'image pour laquelle l'angle de tangage est le plus faible.

L'opération suivante consiste pour l'unité de calcul 250 à déterminer la propension du porteur 1 à détourner le regard en bougeant plutôt la tête 10 ou les yeux 20G, 20D.

Le vendeur explique alors au porteur 1 qu'il doit regarder la première cible supplémentaire 320, puis, dès que celle-ci s'éteint et que la seconde cible supplémentaire 330 s'illumine, qu'il doit détourner rapidement le regard vers cette seconde cible supplémentaire 330.

Dès que le porteur 1 est prêt, le vendeur initie une nouvelle prise de mesures en appuyant sur un bouton ad hoc affiché sur l'écran tactile 270.

L'unité de calcul 250 commande alors l'allumage de la première cible supplémentaire 320 seule, pendant environ 5 secondes, puis l'extinction de la première cible supplémentaire 320 et l'allumage simultané de la seconde cible supplémentaire 330. Au moment de l'extinction de la première cible supplémentaire 320, l'unité de calcul 250 commande l'acquisition et la mémorisation d'une pluralité d'images (par exemple 10) au cours d'une séquence d'environ 1 seconde.

Les images acquises sont alors traitées par l'unité de calcul 250 pour déterminer sur chacune d'entre elles l'angle de lacet $\alpha_i$ de la tête 10 du porteur 1.

Si, sur les 10 images acquises, cet angle varie peu et/ou lentement, l'unité de calcul 250 détermine que le porteur a une propension à détourner le regard en bougeant plutôt les yeux 20G, 20D.

Au contraire, si, sur les 10 images acquises, cet angle varie beaucoup et/ou rapidement, l'unité de calcul 250 détermine que le porteur a une propension à détourner le regard en bougeant plutôt la tête 10.

En variante, l'unité de calcul pourrait procéder autrement.

A titre d'exemple, elle pourrait procéder plus finement, en déterminant également sur chaque image acquise les distances $EG_i$, $ED_i$ séparant les reflets cornéens 162D, 162G de l'axe remarquable A3.

Elle pourrait alors comparer les vitesses et/ou amplitudes de variation de l'angle de lacet $\alpha_i$ avec les vitesses et/ou amplitudes de variation des distances $EG_i$, $ED_i$. Elle pourrait ainsi en déduire si le porteur a une propension à tourner plus rapidement les yeux ou la tête. Elle pourrait même en déduire un coefficient fin, quantifiant la propension du porteur à détourner le regard en bougeant plutôt la tête ou les yeux.

A l'issue de ces différentes opérations, l'unité de calcul 250 a acquis :

- une image scannée des prescriptions du porteur,
- la référence de la monture de lunettes sélectionnée par le porteur 1,
- les demi-écarts pupillaires EG, ED du porteur 1,
- les hauteurs pupillaires HG, HD du porteur 1,
- une image-visible du visage du porteur 1,
- les distances VG2, VD2 séparant les centres de rotation des yeux du porteur 1 et le plan moyen P1 de la monture de lunettes 110, et
- un coefficient quantifiant la propension du porteur 1 à détourner le regard en bougeant plutôt la tête 10 ou les yeux 20G, 20D.

Elle commande alors l'affichage de ces différentes informations sur l'écran tactile 270 pour vérification par le vendeur.

Après validation par le vendeur, l'unité de calcul 250 commande l'impression de ces informations sur une fiche récapitulative. Elle communique par ailleurs ces informations au centre de fabrication des lentilles ophtalmiques.

On pourra éventuellement prévoir que l'unité de calcul 250 communique en outre au centre de fabrication des lentilles ophtalmiques l'ensemble des images acquises.

Alors, le centre de traitement pourra éventuellement réaliser une opération de vérification de toutes les données calculées par l'unité de calcul 250.

Ces données pourront notamment être vérifiées lorsque le logiciel installé dans l'unité de calcul 250 n'aura pas été mis à jour.

Elles pourront également être vérifiées lorsque la monture sélectionnée sera de type « sans cercle » (ou « percée »). En effet, les bords des lentilles de présentation de ces montures sont généralement peu visibles sur les images acquises, ce qui peut générer des erreurs.

Cette opération de vérification pourra être mise en œuvre automatiquement (notamment dans le cas où le logiciel n'est pas à jour), ou manuellement, par un technicien spécialisé qui pourra notamment vérifier que les pupilles et les bords des lentilles de présentation ont bien été repérés.

La présente invention n'est nullement limitée aux modes de réalisation décrits et représentés, mais l'homme du métier saura y apporter toute variante conforme à son esprit.

On pourra en particulier prévoir que le dispositif de mesure se présente, non pas sous la forme d'un kiosque logeant l'unité de calcul, le capteur d'images et les sources de lumière, mais plutôt sous une forme de taille réduite et portable. On pourra ainsi prévoir que l'unité de calcul soit formée par un ordinateur portable dans lequel sera installé un logiciel ad hoc, et dont la caméra (ou « web-cam ») fera office de caméra dans le domaine du visible. Dans cette variante, la caméra infrarouge et la source de lumière seront alors agencées sur un boîtier de petite taille, muni d'une pince de fixation à l'écran de l'ordinateur portable et d'un câble de branchement à l'ordinateur portable. Cette solution sera peu onéreuse et facilement transportable.

Selon une autre variante de réalisation de l'invention, on pourra prévoir que l'unité de calcul se contente d'enregistrer des images et de les communiquer au centre de fabrication de lentilles ophtalmiques, auquel cas ces images seront traitées dans ce centre de fabrication.

**Revendications**

1. Procédé de détermination d'au moins une caractéristique de posture (a) de la tête (10) d'un porteur (1) d'une paire de lunettes (100) comportant une monture (110) et deux lentilles (150G, 150D), à l'aide d'un dispositif de détermination (200) comprenant un capteur d'images (210), au moins une source de lumière (220, 230, 240) et une unité de calcul (250), **caractérisé en ce qu'**il comporte des étapes :

   a) d'acquisition par le capteur d'images (210) d'une image (301, 302, 303) d'une partie au moins de la tête (10) du porteur, sur laquelle apparaît la paire de lunettes (100) éclairée par la source de lumière (220, 230, 240),
   b) de localisation par l'unité de calcul (250), sur ladite image (301, 302, 303), des deux reflets (160D, 160G, 161D, 161G) générés par la source de lumière (220, 230, 240) et réfléchis respectivement par les faces optiques des deux lentilles (150G, 150D) de la paire de lunettes (100) et non par les bords des lentilles ou, si la lentille présente des trous, non pas par les bords desdits trous, et
   c) de déduction par l'unité de calcul (250) de la caractéristique de posture (a) de la tête (10) du porteur (1) par rapport audit capteur d'images (210), en fonction de la position desdits reflets (160D, 160G, 161D, 161G) sur ladite image (301, 302, 303), ladite caractéristique de posture (a) étant un angle de lacet qui sépare le plan sagittal (P2) de la tête (10) du porteur (1) et l'axe optique (A2) du capteur d'images (210).

2. Procédé de détermination selon la revendication précédente, dans lequel, à l'étape b), l'unité de calcul (250) localise également sur ladite image (301, 302, 303) un point ou un axe remarquable (A3) de la monture de lunettes (110), et, à l'étape c), l'unité de calcul (250) déduit la caractéristique de posture (a) en fonction également de la position du point ou de l'axe remarquable (A3) sur ladite image (301, 302, 303).

3. Procédé de détermination selon la revendication précédente, dans lequel l'axe remarquable (A3) est localisé, à l'étape b), en déterminant les positions sur ladite image (301, 302, 303) des cadres circonscrits (151G, 151D) aux lentilles (150G , 150D) ou aux entourages (111G, 111D) de la monture de lunettes (100), et en repérant l'axe moyen passant entre les deux cadres circonscrits (151G, 151D).

4. Procédé de détermination selon la revendication 3, dans lequel, à l'étape c), ledit angle de lacet (a) est calculé en fonction des distances séparant les reflets (160D, 160G, 161D, 161G) de l'axe remarquable (A3).

5. Procédé de détermination selon l'une des revendications précédentes, dans lequel, à l'étape a), le capteur d'images (210) acquiert ladite image (301, 302, 303) dans le domaine infra-rouge.

6. Procédé de détermination selon l'une des revendications précédentes, dans lequel, le dispositif de détermination comportant une source de lumière mobile par rapport audit capteur d'images, préalablement à l'étape a), il est prévu une étape de pré-positionnement de la source de lumière au cours de laquelle l'unité de calcul pilote ladite source de lumière de telle manière que les reflets de ladite source de lumière réfléchie par les deux lentilles de la paire de lunettes soient visibles par le capteur d'images.

7. Méthode d'acquisition utilisant un procédé de détermination conforme à l'une des revendications précédentes pour acquérir des demi-écarts pupillaires (EG, ED) d'un porteur (1) d'une paire de lunettes (100), à l'aide d'un dispositif de détermination (200) comprenant un capteur d'images (210), au moins une source de lumière (220, 230, 240) et une unité de calcul (250), au cours de laquelle la tête (10) du porteur (1) pivote autour de son axe longitudinal (A1) par rapport audit capteur d'images (201), comprenant les opérations :

   i) d'acquisition par le capteur d'images (210) d'au moins deux images (301, 302, 303) distinctes d'au moins une partie de la tête (10) du porteur (1), sur lesquelles apparaissent la paire de lunettes (100) éclairée par la source de lumière (220, 230, 240), pour mettre en œuvre le procédé de détermination conforme à la revendication 5 afin de déterminer l'angle de lacet (a) de la tête (10) du porteur (1) sur chaque image (301, 302, 303) acquise,
   ii) de localisation par l'unité de calcul (250), sur au moins une des images (301, 302, 303) acquises au cours de l'opération i), d'une part, des reflets cornéens (162D, 162G) de la source de lumière (220, 230, 240) réfléchis par les deux cornées du porteur (1), et, d'autre part, d'un axe remarquable (A3) et central de la paire de lunettes (100),
   iii) de mesure par l'unité de calcul (250), sur chaque image traitée au cours de l'opération ii), des distances pupillaires ($ED_{301}$, $EG_{301}$, $ED_{302}$, $EG_{302}$, $ED_{303}$, $EG_{303}$) entre chacun des reflets cornéens (162D, 162G) et l'axe remarquable (A3),
   iv) de déduction des demi-écarts pupillaires (EG, ED) en fonction des distances pupillaires ($ED_{301}$, $EG_{301}$,

$ED_{302}$, $EG_{302}$, $ED_{303}$, $EG_{303}$) mesurées au cours de l'opération iii).

8.  Méthode d'acquisition selon la revendication précédente, dans laquelle l'angle de lacet (a) déterminé sur chaque image (301, 302, 303) acquise au cours de l'opération i) est utilisé :

    - pour sélectionner une unique image (302) à traiter au cours des opérations ii) à iv) dont l'angle de lacet (a) est sensiblement nul, ou
    - pour corriger les distances pupillaires ($ED_{301}$, $EG_{301}$, $ED_{302}$, $EG_{302}$, $ED_{303}$, $EG_{303}$) mesurées sur au moins deux images (301, 302, 303) afin d'en déduire lesdits demi-écarts pupillaires (EG, ED).

9.  Méthode d'acquisition selon l'une des deux revendications précédentes, dans laquelle ladite opération i) comporte une étape de sélection, parmi l'ensemble des images (301, 302, 303) acquises, d'un nombre restreint d'images (301, 302, 303) sur lesquelles les reflets cornéens (162D, 162G) et les reflets (160G, 160D, 161G, 161D) réfléchis par les lentilles (150G, 150D) sont visibles et ne se confondent pas.

10. Méthode d'acquisition selon l'une des trois revendications précédentes, dans laquelle il est prévu une opération de détermination d'une dimension caractéristique de la monture de lunettes (110), pour mettre à l'échelle les distances pupillaires ($ED_{301}$, $EG_{301}$, $ED_{302}$, $EG_{302}$, $ED_{303}$, $EG_{303}$) et/ou les demi-écarts pupillaires (EG, ED).

11. Méthode d'acquisition selon l'une des quatre revendications précédentes, dans laquelle le porteur est immobile et le capteur d'images est piloté pour pivoter autour de l'axe longitudinal de la tête du porteur.

12. Méthode de détection utilisant un procédé de détermination conforme à l'une des revendications 1 à 3 pour détecter le comportement d'un porteur (1) d'une paire de lunettes (100) à l'aide d'un dispositif de détermination (200) comprenant un capteur d'images (210), au moins une source de lumière (220, 230, 240), deux cibles (320, 330) et une unité de calcul (250), comprenant des opérations :

    i) de pré-positionnement du porteur (1) de manière à ce que son regard et sa tête (10) soient dirigés vers l'une des deux cibles (320),
    ii) d'instruction au porteur (1) de détourner rapidement son regard en direction de l'autre des deux cibles (330),
    iii) de détermination d'un angle de lacet ($\alpha_1$) de la tête du porteur en mettant en œuvre un procédé de détermination conforme à la revendication 5, et
    iv) de déduction d'un coefficient quantifiant la propension du porteur à détourner le regard en bougeant plutôt la tête (10) ou les yeux, en fonction de l'angle de lacet ($\alpha_1$) déterminé.

13. Méthode de détection selon la revendication précédente, dans laquelle il est prévu, entre les opérations i) et ii), une opération de détermination d'un premier angle de lacet ($\alpha_0$) de la tête (10) du porteur (1) en mettant en œuvre un procédé de détermination conforme à la revendication 5, et il est prévu, au cours de l'opération iv), de déduire ledit coefficient en fonction également dudit premier angle de lacet ($\alpha_0$).

14. Méthode de détection selon l'une des deux revendications précédentes, dans laquelle, la seconde cible comportant une source lumineuse (330), l'opération ii) consiste à allumer ladite source lumineuse (330).

15. Dispositif de détermination (200) d'au moins une caractéristique de posture (a) de la tête (10) d'un porteur (1) d'une paire de lunettes (100) comportant une monture (110) et deux lentilles (150G, 150D), qui comprend :

    - au moins une source de lumière (220, 230, 240) adaptée à éclairer la tête (10) du porteur (1),
    - un capteur d'images (210) adapté à acquérir une image (301, 302, 303) d'au moins une partie de la tête (10) du porteur (1) sur laquelle apparaît la paire de lunettes (100) éclairée par la source de lumière (220, 230, 240), et
    - une unité de calcul (250) adaptée à traiter ladite image (301, 302, 303), **caractérisé en ce que** ladite unité de calcul (250) est adaptée à :
    - localiser sur ladite image (301, 302, 303) les reflets (160G, 160D, 161G, 161D) de la source de lumière (220, 230, 240) réfléchis par les faces avant des deux lentilles (150G, 150D) et non par les bords des lentilles ou, si la lentille présente des trous, par les bords desdits trous, et à
    - en déduire ladite caractéristique de posture ($\alpha$), ladite caractéristique de posture (a) étant un angle de lacet qui sépare le plan sagittal (P2) de la tête (10) du porteur (1) et l'axe optique (A2) du capteur d'images (210).

**Patentansprüche**

1. Verfahren zur Bestimmung mindestens eines Haltungsmerkmals (a) des Kopfes (10) eines Trägers (1) einer Brille (100), die ein Gestell (110) und zwei Linsen (150G, 150D) aufweist, mit Hilfe einer einen Bildsensor (210), mindestens eine Lichtquelle (220, 230, 240) und eine Recheneinheit (250) enthaltenden Bestimmungsvorrichtung (200), **dadurch gekennzeichnet, dass** es Schritte aufweist:

   a) der Erfassung eines Bilds (301, 302, 303) mindestens eines Teils des Kopfes (10) des Trägers, auf dem die von der Lichtquelle (220, 230, 240) beleuchtete Brille (100) erscheint, durch den Bildsensor (210),
   b) der Lokalisierung der zwei Lichtreflexe (160D, 160G, 161D, 161G), die von der Lichtquelle (220, 230, 240) erzeugt und je von den optischen Flächen der zwei Linsen (150G, 150D) der Brille (100) und nicht von den Rändern der Linsen oder, wenn die Linse Löcher aufweist, nicht von den Rändern der Löcher reflektiert werden, auf dem Bild (301, 302, 303) durch die Recheneinheit (250), und
   c) der Ableitung des Haltungsmerkmals (a) des Kopfes (10) des Trägers (1) bezüglich des Bildsensors (210) durch die Recheneinheit (250) abhängig von der Position der Lichtreflexe (160D, 160G, 161D, 161G) auf dem Bild (301, 302, 303), wobei das Haltungsmerkmal (a) ein Gierwinkel ist, der die Sagittalebene (P2) des Kopfes (10) des Trägers (1) und die optische Achse (A2) des Bildsensors (210) trennt.

2. Bestimmungsverfahren nach dem vorhergehenden Anspruch, wobei die Recheneinheit (250) im Schritt b) ebenfalls einen Punkt oder eine beachtenswerte Achse (A3) des Brillengestells (110) auf dem Bild (301, 302, 303) lokalisiert, und die Recheneinheit (250) im Schritt c) das Haltungsmerkmal (a) ebenfalls abhängig von der Position des Punkts oder der beachtenswerten Achse (A3) auf dem Bild (301, 302, 303) ableitet.

3. Bestimmungsverfahren nach dem vorhergehenden Anspruch, wobei die beachtenswerte Achse (A3) im Schritt b) lokalisiert wird, indem die Positionen der auf die Linsen (150G, 150D) oder die Einfassungen (111G, 111D) des Brillengestells (100) beschränkten Rahmen (151G, 151D) auf dem Bild (301, 302, 303) bestimmt werden, und indem die zwischen den zwei beschränkten Rahmen (151G, 151D) verlaufende Mittelachse markiert wird.

4. Bestimmungsverfahren nach Anspruch 3, wobei der Gierwinkel (a) im Schritt c) abhängig von den die Lichtreflexe (160D, 160G, 161D, 161G) von der beachtenswerten Achse (A3) trennenden Abständen berechnet wird.

5. Bestimmungsverfahren nach einem der vorhergehenden Ansprüche, wobei der Bildsensor (210) im Schritt a) das Bild (301, 302, 303) im Infrarotbereich erfasst.

6. Bestimmungsverfahren nach einem der vorhergehenden Ansprüche, wobei, da die Bestimmungsvorrichtung eine bezüglich des Bildsensors bewegliche Lichtquelle aufweist, vor dem Schritt a) ein Schritt der Vorpositionierung der Lichtquelle vorgesehen ist, während dessen die Recheneinheit die Lichtquelle so steuert, dass die Lichtreflexe der von den zwei Linsen der Brille reflektierten Lichtquelle für den Bildsensor sichtbar sind.

7. Erfassungsverfahren, das ein Bestimmungsverfahren gemäß einem der vorhergehenden Ansprüche verwendet, um Pupillen-Halbabstände (EG, ED) eines Trägers (1) einer Brille (100) mit Hilfe einer Bestimmungsvorrichtung (200) zu erfassen, die einen Bildsensor (210), mindestens eine Lichtquelle (220, 230, 240) und eine Recheneinheit (250) enthält, während dessen der Kopf (10) des Trägers (1) bezüglich des Bildsensors (201) um seine Längsachse (A1) schwenkt, das die Vorgänge enthält:

   i) der Erfassung von mindestens zwei unterschiedlichen Bildern (301, 302, 303) mindestens eines Teils des Kopfes (10) des Trägers (1) durch den Bildsensor (210), auf denen die von der Lichtquelle (220, 230, 240) beleuchteten Brillen (100) erscheinen, um das Bestimmungsverfahren gemäß Anspruch 5 durchzuführen, um den Gierwinkel (a) des Kopfes (10) des Trägers (1) auf jedem erfassten Bild (301, 302, 303) zu bestimmen,
   ii) der Lokalisierung auf mindestens einem der während des Vorgangs (i) erfassten Bilder (301, 302, 303), einerseits der Hornhautreflexe (162D, 162G) der Lichtquelle (220, 230, 240), die von den zwei Hornhäuten des Trägers (1) reflektiert werden, und andererseits einer beachtenswerten und mittleren Achse (A3) der Brille (100) durch die Recheneinheit (250),
   iii) der Messung auf jedem während des Vorgangs ii) behandelten Bild der Pupillenabstände ($ED_{301}$, $EG_{301}$, $ED_{302}$, $EG_{302}$, $ED_{303}$, $EG_{303}$) zwischen jedem der Hornhautreflexe (162D, 162G) und der beachtenswerten Achse (A3) durch die Recheneinheit (250),
   iv) der Ableitung der Pupillen-Halbabstände (EG, ED) abhängig von den während des Vorgangs iii) gemessenen Pupillenabständen ($ED_{301}$, $EG_{301}$, $ED_{302}$, $EG_{302}$, $ED_{303}$, $EG_{303}$).

8. Erfassungsverfahren nach dem vorhergehenden Anspruch, wobei der auf jedem während des Vorgangs i) erfassten Bild (301, 302, 303) bestimmte Gierwinkel ($\alpha$) verwendet wird:

   - um ein einziges während der Vorgänge ii) bis iv) zu behandelndes Bild (302) auszuwählen, dessen Gierwinkel (a) im Wesentlichen null ist, oder
   - um die an mindestens zwei Bildern (301, 302, 303) gemessenen Pupillenabstände ($ED_{301}$, $EG_{301}$, $ED_{302}$, $EG_{302}$, $ED_{303}$, $EG_{303}$) zu korrigieren, um daraus die Pupillen-Halbabstände (EG, ED) abzuleiten.

9. Erfassungsverfahren nach einem der zwei vorhergehenden Ansprüche, wobei der Vorgang i) einen Schritt der Auswahl, unter der Gesamtheit der erfassten Bilder (301, 302, 303), einer begrenzten Anzahl von Bildern (301, 302, 303) aufweist, auf denen die Hornhautreflexe (162D, 162G) und die von den Linsen (150G, 150D) reflektierten Lichtreflexe (160G, 160D, 161G, 161D) sichtbar sind und nicht ineinander übergehen.

10. Erfassungsverfahren nach einem der drei vorhergehenden Ansprüche, wobei ein Vorgang der Bestimmung einer charakteristischen Abmessung des Brillengestells (110) vorgesehen ist, um die Pupillenabstände ($ED_{301}$, $EG_{301}$, $ED_{302}$, $EG_{302}$, $ED_{303}$, $EG_{303}$) und/oder die Pupillen-Halbabstände (EG, ED) zu skalieren.

11. Erfassungsverfahren nach einem der vier vorhergehenden Ansprüche, wobei der Träger unbeweglich ist und der Bildsensor gesteuert wird, um um die Längsachse des Kopfes des Trägers zu schwenken.

12. Erkennungsverfahren, das ein Bestimmungsverfahren gemäß einem der Ansprüche 1 bis 3 verwendet, um das Verhalten eines Trägers (1) einer Brille (100) mit Hilfe einer Bestimmungsvorrichtung (200) zu erkennen, die einen Bildsensor (210), mindestens eine Lichtquelle (220, 230, 240), zwei Ziele (320, 330) und eine Recheneinheit (250) aufweist, das Vorgänge enthält:

   i) der Vorpositionierung des Trägers (1) derart, dass sein Blick und sein Kopf (10) zu einem der zwei Ziele (320) gerichtet sind,
   ii) der Anweisung des Trägers (1), seinen Blick schnell in Richtung des anderen der zwei Ziele (330) umzulenken,
   iii) der Bestimmung eines Gierwinkels ($\alpha_1$) des Kopfes des Trägers unter Anwendung eines Bestimmungsverfahrens gemäß Anspruch 5, und
   iv) der Ableitung eines Koeffizienten, der die Bereitschaft des Trägers, den Blick umzulenken, indem er eher den Kopf (10) oder die Augen bewegt, abhängig vom bestimmten Gierwinkel ($\alpha_1$) quantifiziert.

13. Erkennungsverfahren nach dem vorhergehenden Anspruch, wobei zwischen den Vorgängen i) und ii) ein Vorgang der Bestimmung eines ersten Gierwinkels ($\alpha_0$) des Kopfes (10) des Trägers (1) vorgesehen ist, indem ein Bestimmungsverfahren gemäß Anspruch 5 angewendet wird, und während des Vorgangs iv) vorgesehen ist, den Koeffizienten ebenfalls abhängig vom ersten Gierwinkel ($\alpha_0$) abzuleiten.

14. Erkennungsverfahren nach einem der zwei vorhergehenden Ansprüche, wobei, da das zweite Ziel eine Lichtquelle (330) aufweist, der Vorgang ii) darin besteht, die Lichtquelle (330) einzuschalten.

15. Vorrichtung (200) zur Bestimmung mindestens eines Haltungsmerkmals (a) des Kopfes (10) eines Trägers (1) einer ein Gestell (110) und zwei Linsen (150G, 150D) aufweisenden Brille (100), die enthält:

   - mindestens eine Lichtquelle (220, 230, 240), die geeignet ist, den Kopf (10) des Trägers (1) zu beleuchten,
   - einen Bildsensor (210), der geeignet ist, ein Bild (301, 302, 303) mindestens eines Teils des Kopfes (10) des Trägers (1) zu erfassen, auf dem die von der Lichtquelle (220, 230, 240) beleuchtete Brille (100) erscheint, und
   - eine Recheneinheit (250), die geeignet ist, das Bild (301, 302, 303) zu behandeln,

   **dadurch gekennzeichnet, dass** die Recheneinheit (250) geeignet ist:

   - auf dem Bild (301, 302, 303) die Lichtreflexe (160G, 160D, 161G, 161D) der Lichtquelle (220, 230, 240) zu lokalisieren, die von den Vorderflächen der zwei Linsen (150G, 150D) und nicht von den Rändern der Linsen oder, wenn die Linse Löcher aufweist, von den Rändern der Löcher reflektiert werden, und
   - daraus das Haltungsmerkmal (a) abzuleiten, wobei das Haltungsmerkmal (a) ein Gierwinkel ist, der die Sagittalebene (P2) des Kopfes (10) des Trägers (1) und die optische Achse (A2) des Bildsensors (210) trennt.

**Claims**

1. A method for determining at least one postural characteristic ($\alpha$) of the head (10) of a wearer (1) of a pair of spectacles (100) comprising a frame (110) and two lenses (150G, 150D), using a determining device (200) comprising an image sensor (210), at least one light source (220, 230, 240) and a processing unit (250), **characterized in that** it comprises steps of:

   a) acquiring via the image sensor (210) an image (301, 302, 303) of at least part of the head (10) of the wearer, in which image appears the pair of spectacles (100) illuminated by the light source (220, 230, 240);
   b) locating via the processing unit (250), in said image (301, 302, 303), two reflections (160D, 160G, 161D, 161G) generated by the light source (220, 230, 240) and respectively reflected by the optical faces of the two lenses (150G, 150D) of the pair of spectacles (100) and not by the edges of the lenses or by the edges of the openings in the lenses if any; and
   c) deducing via the processing unit (250) the postural characteristic ($\alpha$) of the head (10) of the wearer (1) relative to said image sensor (210), as a function of the position of said reflections (160D, 160G, 161D, 161G) in said image (301, 302, 303), said postural characteristic ($\alpha$) being a yaw angle ($\alpha$) which separates the sagittal plane (P2) of the head (10) of the wearer (1) and the optical axis (A2) of the image sensor (210).

2. The determining method as claimed in the preceding claim, in which, in step b), the processing unit (250) also locates in said image (301, 302, 303) a point or an observable axis (A3) of the spectacle frame (110), and, in step c), the processing unit (250) deduces the postural characteristic ($\alpha$) also as a function of the position of the point or the observable axis (A3) in said image (301, 302, 303).

3. The determining method as claimed in the preceding claim, in which the observable axis (A3) is located, in step b), by determining the positions in said image (301, 302, 303) of boxes (151G, 151D) circumscribed around the lenses (150G , 150D) or rims (111G, 111D) of the spectacle frame (100), and by locating the midline passing between the two circumscribed boxes (151G, 151D).

4. The determining method as claimed in claim 3, in which, in step c), said yaw angle ($\alpha$) is calculated as a function of the distances separating the reflections (160D, 160G, 161D, 161G) from the observable axis (A3).

5. The determining method as claimed in one of the preceding claims, in which, in step a), the image sensor (210) acquires said image (301, 302, 303) in the infrared domain.

6. The determining method as claimed in one of the preceding claims, in which, the determining device comprising a light source that is movable relative to said image sensor, provision is made, before step a), for a step of pre-positioning the light source, in which step the processing unit moves said light source so that the reflections of said light source reflected by the two lenses of the pair of spectacles are visible to the image sensor.

7. A procedure using a determining process according to any preceding claim, for acquiring the half pupillary distances (EG, ED) of a wearer (1) of a pair of spectacles (100), using a determining device (200) comprising an image sensor (210), at least one light source (220, 230, 240) and a processing unit (250), in which the head (10) of the wearer (1) pivots about its longitudinal axis (A1) relative to said image sensor (201), said procedure comprising operations of:

   i) acquiring via the image sensor (210) at least two separate images (301, 302, 303) of at least one part of the head (10) of the wearer (1), in which images the pair of spectacles (100) illuminated by the light source (220, 230, 240) appear, in order to implement the determining method as claimed in claim 5 so as to determine the yaw angle ($\alpha$) of the head (10) of the wearer (1) in each acquired image (301, 302, 303);
   ii) locating via the processing unit (250), in at least one of the images (301, 302, 303) acquired in operation i), on the one hand, corneal reflections (162D, 162G) of the light source (220, 230, 240) reflected by the two corneas of the wearer (1), and, on the other hand, an observable and central axis (A3) of the pair of spectacles (100);
   iii) measuring via the processing unit (250), in each image processed in operation ii), pupillary distances ($ED_{301}$, $EG_{301}$, $ED_{302}$, $EG_{302}$, $ED_{303}$, $EG_{303}$) between each of the corneal reflections (162D, 162G) and the observable axis (A3); and
   iv) deducing half pupillary distances (EG, ED) as a function of the pupillary distances ($ED_{301}$, $EG_{301}$, $ED_{302}$, $EG_{302}$, $ED_{303}$, $EG_{303}$) measured in operation iii).

8. The acquiring procedure as claimed in the preceding claim, in which the yaw angle ($\alpha$) determined in each image (301, 302, 303) acquired in operation i) is used:

- to select a single image (302) to be processed in operations ii) to iv), the yaw angle ($\alpha$) of which is substantially zero; or
- to correct the pupillary distances ($ED_{301}$, $EG_{301}$, $ED_{302}$, $EG_{302}$, $ED_{303}$, $EG_{303}$) measured in at least two images (301, 302, 303) in order to deduce therefrom said half pupillary distances (EG, ED).

9. The acquiring procedure as claimed in one of the two preceding claims, in which said operation i) comprises a step of selecting, from all of the acquired images (301, 302, 303), a small number of images (301, 302, 303) in which the corneal reflections (162D, 162G) and the reflections (160G, 160D, 161G, 161D) reflected by the lenses (150G, 150D) are visible and do not overlap.

10. The acquiring procedure as claimed in one of the three preceding claims, in which provision is made for an operation for determining a characteristic dimension of the spectacle frame (110), in order to scale the pupillary distances ($ED_{301}$, $EG_{301}$, $ED_{302}$, $EG_{302}$, $ED_{303}$, $EG_{303}$) and/or the half pupillary distances (EG, ED).

11. The acquiring procedure as claimed in one of the four preceding claims, in which the wearer stays still and the image sensor is moved to pivot about the longitudinal axis of the head of the wearer.

12. A procedure using a determining process according to any one of claims 1 to 3, for detecting the behavior of a wearer (1) of a pair of spectacles (100) using a determining device (200) comprising an image sensor (210), at least one light source (220, 230, 240), two targets (320, 330) and a processing unit (250), comprising operations of:

i) pre-positioning the wearer (1) so that their gaze and their head (10) are directed toward one of the two targets (320);
ii) instructing the wearer (1) to rapidly turn their gaze in the direction of the other of the two targets (330);
iii) determining a yaw angle ($\alpha_1$) of the head of the wearer by implementing a determining method as claimed in claim 5; and
iv) deducing a coefficient quantifying the propensity of the wearer to turn their gaze by moving rather their head (10) or their eyes, as a function of the determined yaw angle ($\alpha_1$)

13. The detecting procedure as claimed in the preceding claim, in which provision is made, between operations i) and ii), for an operation for determining a first yaw angle ($\alpha_0$) of the head (10) of the wearer (1) by implementing a determining method as claimed in claim 1, and provision is made, in operation iv), to deduce said coefficient also as a function of said first yaw angle ($\alpha_0$).

14. The detecting procedure as claimed in one of the two preceding claims, in which, the second target comprising a light source (330), operation ii) consists in turning on said light source (330).

15. A device (200) for determining at least one postural characteristic ($\alpha$) of the head (10) of a wearer (1) of a pair of spectacles (100) comprising a frame (110) and two lenses (150G, 150D), which comprises:

- at least one light source (220, 230, 240) suitable for illuminating the head (10) of the wearer (1);
- an image sensor (210) suitable for acquiring an image (301, 302, 303) of at least one part of the head (10) of the wearer (1), in which image the pair of spectacles (100) illuminated by the light source (220, 230, 240) appears; and
- a processing unit (250) suitable for processing said image (301, 302, 303),

**characterized in that** said processing unit (250) is suitable for:

- locating on said image (301, 302, 303) the reflections (160G, 160D, 161G, 161D) of the light source (220, 230, 240) reflected by the front faces of the two lenses (150G, 150D) and not by the edges of the lenses or by the edges of the openings in the lenses if any, and
- deducing therefrom said postural characteristic ($\alpha$), said postural characteristic ($\alpha$) being a yaw angle ($\alpha$) which separates the sagittal plane (P2) of the head (10) of the wearer (1) and the optical axis (A2) of the image sensor (210).

Fig.1

Fig.2

Fig.3

Fig.4

Fig.5

Fig.6

Fig.7

Fig.8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008132356 A
- WO 2008009423 A

- DE 102009004383